# EUROPEAN PATENT SPECIFICATION

(11) **EP 1 789 540 B1**
(45) Date of publication and mention of the grant of the patent: **18.05.2011**
(21) Application number: 05791447.5
(22) Date of filing: 24.08.2005
(51) Int. Cl.: C12N 5/074

(54) **NON-EMBRYONIC TOTIPOTENT BLASTOMER-LIKE STEM CELLS AND METHODS THEREFOR**
BLASTOMERENÄHNLICHE, NICHTEMBRYONALE, TOTIPOTENTE STAMMZELLEN UND METHODEN ZUR GEWINNUNG UND ANWENDUNG
CELLULES SOUCHES DE TYPE BLASTOMERES TOTIPOTENTES NON EMBRYONNAIRES ET PROCEDES ASSOCIES

(30) Priority: 03.09.2004 US 606913 P; 08.09.2004 US 607624 P
(43) Date of publication of application: 30.05.2007
(73) Proprietor: Moraga Biotechnology Inc., Los Angeles, CA 90049 (US)
(72) Inventor: YOUNG, Henry E., Macon, GA 31207 (US); BLACK, JR., Asa Calvin, Macon, GA 31211 (US)
(74) Representative: Rupp, Christian
(86) International application number: PCT/US2005/030284
(87) International publication number: WO 2006/028723

(56) References cited:
- WO-A-01/11011
- WO-A-02/31123
- WO-A-02/064748
- YOUNG HENRY E ET AL: "Adult reserve stem cells and their potential for tissue engineering." CELL BIOCHEMISTRY AND BIOPHYSICS, vol. 40, no. 1, February 2004 (2004-02), pages 1-80, XP002354278 ISSN: 1085-9195
- VERFAILLIE C M: "Adult stem cells: Assessing the case for pluripotency" TRENDS IN CELL BIOLOGY, ELSEVIER SCIENCE LTD, vol. 12, no. 11, November 2002 (2002-11), pages 502-508, XP002268623 ISSN: 0962-8924
- JIANG YUEHUA ET AL: "Multipotent progenitor cells can be isolated from postnatal murine bone marrow, muscle, and brain" EXPERIMENTAL HEMATOLOGY (CHARLOTTESVILLE), vol. 30, no. 8, August 2002 (2002-08), pages 896-904, XP002354279 ISSN: 0301-472X
- HOWELL JONATHAN C ET AL: "Pluripotent stem cells identified in multiple murine tissues." HEMATOPOIETIC STEM CELLS 2002: GENETICS AND FUNCTION NEW YORK ACADEMY OF SCIENCES, 2 EAST 63RD STREET, NEW YORK, NY, 10021, USA SERIES : ANNALS OF THE NEW YORK ACADEMY OF SCIENCES (ISSN 0077-8923 (ISSN PRINT)), 2003, pages 158-173, XP002354280 & FOURTH INTERNATIONAL SYMPOSIUM ON HEMATOPOIETIC STEM CELLS; TUEBINGEN, GERMANY; SEPTEMBER 19-21, 2002 ISSN: 1-57331-466-8

## Description

This application claims priority to our copending U.S. provisional patent applications with the serial numbers 60/606,913, filed September 3, 2004 and 60/607,624, filed September 8, 2004.

### Field of The Invention

The field of the invention is stem cells and reagents for same, and especially as they relate to totipotent non-embryonic stem cells.

### Background of The Invention

### Stem Cells

It is currently thought that mammalian cells progress from embryonic cell stages to fully developed cells through a sequence of totipotent blastomeric cells that develop into pluripotent epiblastic cells, which develop into germ layer lineage cells, which give rise to multipotent progenitor cells that develop to tripotent, then bipotent, then unipotent progenitor cells and finally to the differentiated cell types.

Remarkably, while the vast majority of cells progresses through that sequence of development and differentiation, a few cells become reserve precursor cells that provide for continual maintenance and repair of the organism. Known reserve precursor cells located within the postnatal individual include epiblast-like stem cells, germ layer lineage stem cells (ectodermal germ layer lineage stem cells, endodermal, germ layer lineage stem cells, and the mesodermal germ layer lineage stem cells), and various progenitor cells. In recent years, particular interest focused on early-stage cells, and especially embryonic stem cells.

Embryonic stem cells (ESC) are uncommitted cells isolated from embryonic tissues. For example, ESC have been isolated from the blastocyst, inner cell mass, and gonadal ridges of mouse, rabbit, rat, pig, sheep, primate, and human embryos (Evans and Kauffman, 1981; Iannaccone et al., 1994; Graves and Moreadith, 1993; Martin, 1981; Notarianni et al., 1991; Thomson, et al., 1995; Thomson, et al., 1998; Shamblott, et al., 1998). When injected into embryos, ESC can give rise to all somatic lineages as well as functional gametes (i.e., sperm). ESC typically spontaneously differentiate in serum-free defined medium in the absence of agents that inhibit differentiation (e.g., leukemia inhibitory factor). Further known embryonic stem cell preparations from embryoid tissue, post-morula tissue, blastocyst stage and pre-blastocyst stage were described in U.S. Pat. App. No. 2003/0175955, EP 1 176 189, WO 1997/020035, and WO 1995/016770, respectively. However, such cell preparations are either pluripotent and/or isolated from an embryo, which is ethically controversial. Totipotent bovine embryonic stem cells have been reported in U.S. Pat. No. 6,107,543, and ungulate germ-line forming stem cells (possibly not totipotent) have been described in U.S. Pat. No. 6,703,209.

In still further known methods, pluripotent stem cells have been isolated from non-embryonic sources, including from umbilical cord matrix as described in U.S. Pat. App. No. 2003/0161818 and postnatal gonadal tissue as taught in WO 2002/031123. However, while such cells do not require destruction of an embryo and are therefore potentially of interest for human stem cells, the so isolated stem cells have not been demonstrated to be totipotent.

Upon differentiation *in vitro* all or almost all of these cells express a wide variety of cell types, including gametes, as well as cells derived from the ectodermal, mesoderm, and endodermal germ layer lineages. Unfortunately, when currently known uncommitted embryonic stem cells are implanted into animals, they typically spontaneously differentiate *in situ,* forming teratomas. These tumors contain various types of cells and tissue derived from all three primary germ layer lineages (Thomson et al., 1988). Therefore, while ESC appear to have therapeutic potential in transplantation therapies, their tendency to differentiate spontaneously in an uncontrolled manner places limitations on their usefulness.

### Stem Cell Propagation

Growth medium for most stem cells grown in culture is routinely supplemented with animal and/or human serum to optimize and enhance cell viability. The constituents of serum include water, amino acids, glucose, albumins, immunoglobulins, and one or more bioactive agents. Potential bioactive agents present in serum include agents that induce proliferation, agents that accelerate phenotypic expression, agents that induce differentiation, agents that inhibit proliferation, agents that inhibit phenotypic expression, and agents that inhibit differentiation. Unfortunately, the identity(ies), concentration(s), and potential combinations of specific bioactive agents contained in different lots of serum is/are unknown. One or more of these unknown agents in serum have shown a negative impact on the isolation, cultivation, cryopreservation, and purification of lineage-uncommitted blastomere-like stem cells. Similarly, where feeder layers for stem cells were employed, contamination of stem cell cultures with feeder layer specific components, and especially viruses frequently occurs.

Alternatively, serum-free media are known for general cell culture, and selected pluripotent stem cells have been propagated in such medium containing a plurality of growth factors as described in US20050164380, US20030073234, US6617159, US6117675, or EP1298202

Thus, while numerous compositions and methods for stem cells are known in the art, all or almost all of them suffer from one or more disadvantages. Therefore, there is still a need for improved stem cells, compositions, and reagents for their production, maintenance, and differentiation, and especially for postnatal totipotent blastomere-like stem cells.

### Summary of the Invention

The present invention is directed to compositions and method related to blastomere-like totipotent stem cells that express telomerase and carry surface markers CEA-CAM-1⁺, SSEA-1⁻, SSEA-3⁻, SSEA-4⁻, and optionally CD10⁻, and CD66e⁺ (e.g., where the cell is from a human)

In one aspect of the inventive subject matter, an isolated stem cell is preferably a mammalian, post-natal, totipotent stem cell having surface markers CD66e⁺, CEA-CAM-1⁺, CD10⁻, SSEA-1⁻, SSEA-3⁻, and SSEA-4⁻. Such cells advantageously differentiate into a placental cell or a germ cell upon stimulation with a differentiating medium, and are known to undergo at least 100, more typically at least 200, and most typically at least 300 doublings while maintaining totipotent character in serum-free medium in the absence of differentiation inhibitors. Thus, cells according to the inventive subject matter will typically not spontaneously differentiate in defined serum-free medium in the absence of differentiation inhibitors, will remain quiescent and do not form a cancerous tissue when implanted into an animal.

Such cells may further be characterized by expression of Oct-3/4, Nanog, Nanos, BMI-1, IDE1, IDE3, ABCG2, CXCR-4, and/or BCL-2, and lack of expression of CD1 a, CD2, CD3, CD4, CD5, CD7, CDB, CD9, CD11 b, CD11 c, CD13, CD14, CD 15, CD16, CD18, CD19, CD20, CD22, CD23, CD24, CD25, CD31, CD33, CD34, CD36, CD38, CD41, CD42b, CD45, CD49d, CD55, CD56, CD57, CD59, CD61, CD62E, CD65, CD68, CD69, CD71, CD79, CD83, CD90, CD95, CD105, CD106, CD 117, CD123, CD135, CD166, Glycophorin-A, MHC-I, HLA-DRII, FMC-7, Annexin-V, and/or LIN

In still further contemplated aspects, the inventors contemplate a method of isolating a stem cell in which a plurality of cells is received from a mammalian tissue. The cells are then cultivated past confluence to obtain multiple layers and collected. In a further step, the collected cells are slow-frozen to a temperature of higher than -100 °C for at least 12 hours and thawed thereafter. In a still further step, germ line layer stem cells and epiblast-like stem cells are removed from the thawed cells using cell surface markers to form a cell suspension such that the suspension is enriched in stem cells having surface markers CEA-CAM-1⁺, SSEA-1⁻, SSEA-3⁻, SSEA-4⁻, and optionally CD66e⁺, and CD10⁻. Most preferably, the mammalian tissue is human connective tissue. Furthermore, it is generally preferred that the germ layer lineage stem cells are removed using antibodies specific to at least one of CD13 and CD90, and that the epiblast-like stem cells are removed using antibodies specific to at least one of CD10, SSEA-1, SSEA-3, and SSEA-4.

In further contemplated aspects, the inventors contemplate method of regenerating tissue in a mammal in which contemplated stem cells are provided. In another step, the stem cells are implanted into the mammal. Where desirable, the stem cell can be induced *in vitro* to differentiate to an epiblast-like stem cell before the step of implanting, or can be induced *in vitro* to differentiate to an ectodermal cell, an endodermal cell, or a mesodermal cell, or can be induced *in vitro* to differentiate into multipotent, tripotent, bipotent, or unipotent progenitor cells before the step of implanting. Typically, implantation will be into a tissue undergoing repair.

Various objects, features, aspects and advantages of the present invention will become more apparent from the following detailed description of preferred embodiments of the invention.

### Brief Description of the Drawings

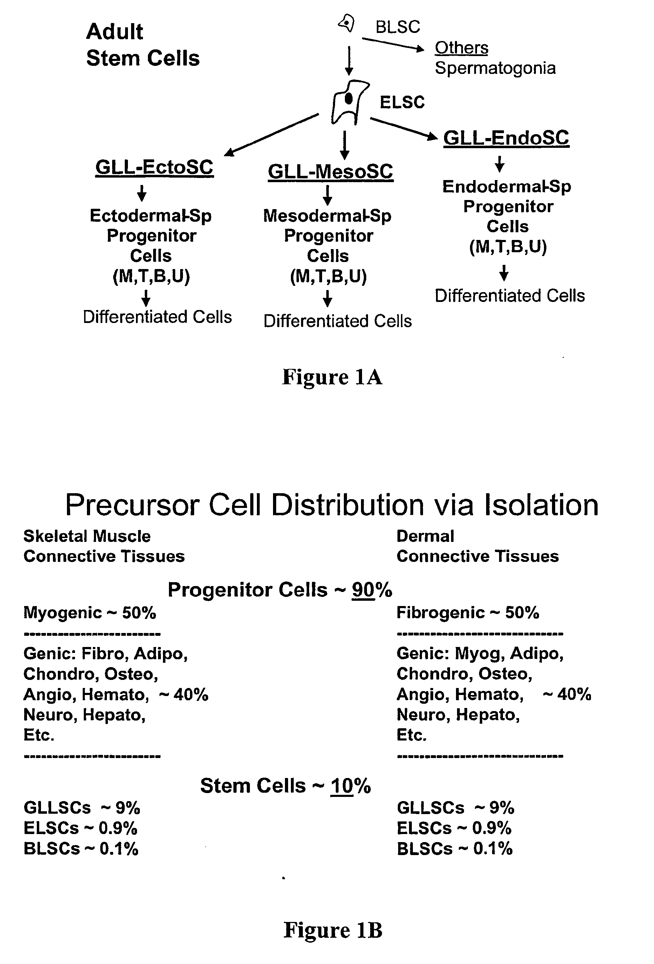

Figure 1A is a schematic representation of exemplary uses of contemplated stem cells.
Figure 1B is a schematic representation of stem cell distribution in selected tissues.

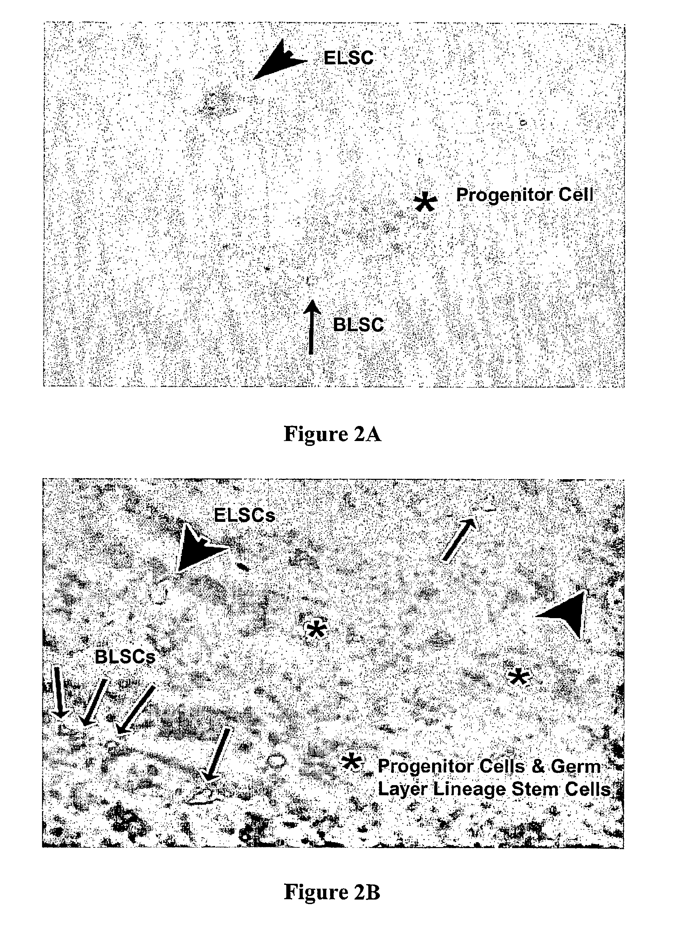

Figure 2A is a micrograph depicting a blastomere-like stem cell (BLSC), an epiblast-like stem cell (ELSC), and a progenitor cell.
Figure 2B is a micrograph depicting blastomere-like stem cells (BLSC) during one step of the isolation process.

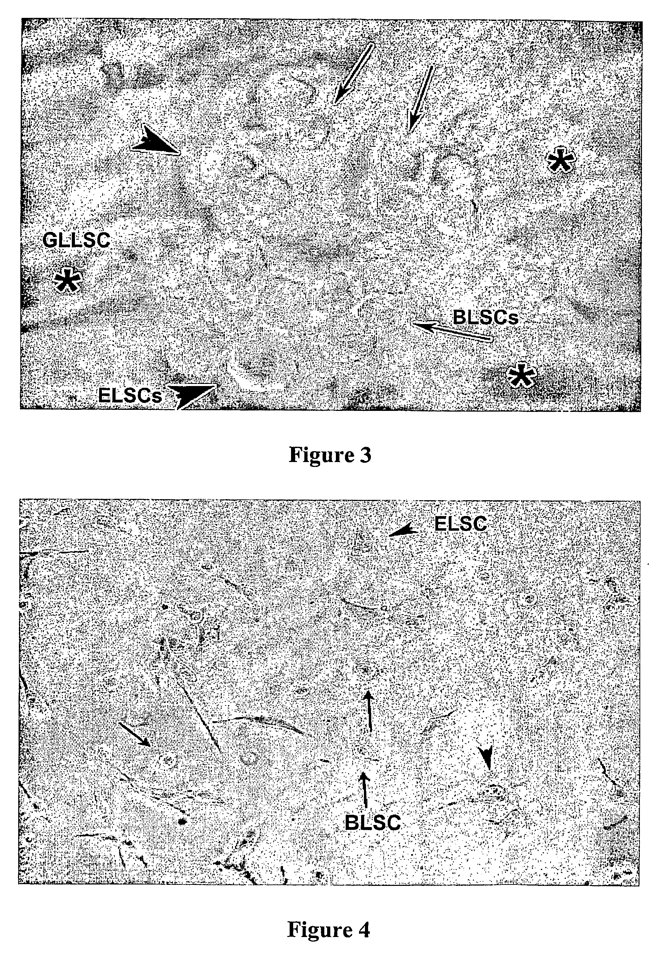

Figure 3 is a micrograph depicting blastomere-like stem cells (BLSC) during another step of the isolation process.
Figure 4 is a micrograph depicting blastomere-like stem cells (BLSC) during a further step of the isolation process.

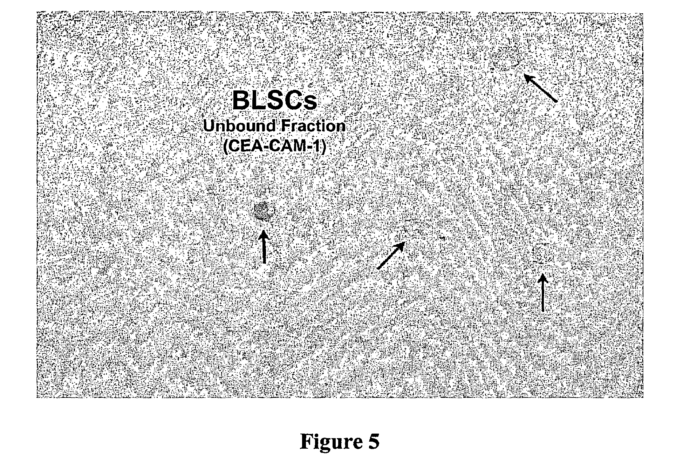

Figure 5 is a micrograph depicting isolated blastomere-like stem cells (BLSC) after the final step in the isolation process.

### Detailed Description

The inventors have unexpectedly discovered that totipotent stem cells can be obtained from a mammal, and particularly from human, wherein such stem cells have blastomere-like character and wherein the stem cells are isolated from a portion (e.g., biopsy) of the mammal or human without killing the mammal or human. Typically, such blastomere-like stem cells are isolated from connective tissue of a post-natal (most typically adult) mammal/human and are less than 1 µm in size in the unfixed state. It should be particularly appreciated that the stem cells according to the inventive subject matter can give rise to germ line progeny, including spermatogonia.

The term "post-natal" as used herein refers to a stage in development of an organism after birth (which may also include premature birth (i.e., at least 60% of normal gestation)). Most typically post-natal stem cells according to the inventive subject matter are isolated from an adult, but earlier stages (e.g., prepubescent or infant stages) are also deemed suitable. Furthermore, the term "totipotent" as used herein in conjunction with a cell refers to a pluripotent cell that also has the ability to give rise to placental and/or gametes.

Remarkably, the blastomere-like stem cells (BLSC) derived from post-natal, rather than embryonic tissues are not committed to any tissue lineage and are of normal karyotype. Contemplated cells typically express Oct-3/4, Nanog, Nanos, BMI-1, IDE1, IDE3, ABCG2, CXCR-4, BCL-2, CEA-CAM-1, and/or the CD66e cell surface marker. In contrast, BLSC typically do not express stage-specific embryonic antigens SSEA-1, SSEA-3, or SSEA-4, and commonly fail to express CD1 a, CD2, CD3, CD4, CD5, CD7, CDB, CD9, CD10, CD11 b, CD11 c, CD 13, CD 14, CD15, CD16, CD 18, CD 19, CD20, CD22, CD23, CD24, CD25, CD31, CD33, CD34, CD36, CD38, CD41, CD42b, CD45, CD49d, CD55, CD56, CD57, CD59, CD61, CD62E, CD65, CD68, CD69, CD71, CD79, CD83, CD90, CD95, CD105, CD106, CD117, CD123, CD135, CD166, Glycophorin-A, MHC-I, HLA-DRII, FMC-7, Annexin-V, and/or LIN cell surface markers.

It should be especially appreciated that the BLSC according to the inventive subject matter remain quiescent in serum-free defined medium in the absence of differentiation inhibitory agents (e.g., leukemia inhibitory factor, or anti-differentiation factor), and when implanted into animals do not form cancerous tissues. In contrast, implanted BLSC remain quiescent after implantation or incorporate into all tissues undergoing repair.

It should be further noted that the BLSC presented herein can also be stimulated *in vitro* to proliferate (most typically in response to one or more growth factors). Remarkably, when stimulated, post-natal blastomere-like stem cells exhibit extended self-renewal as long as they remain lineage-uncommitted. Furthermore, the BLSC are not contact inhibited at confluence and demonstrate telomerase activity.

The inventors further discovered (see experimental data) that post-natal BLSC have the ability to generate all tissues of the conceptus, including embryonic/fetal portions of the placenta, germ cells, and all somatic cells of the embryo/fetus from all three germ layer lineages. For example, a BLSC cell line and a post-natal BLSC rat clone were both induced after more than 300 doublings to form spermatogonia and somatic cells of the embryo. The somatic cells included pluripotent epiblast-like stem cells, germ layer lineage stem cells, lineage-committed progenitor cells, and differentiated cells.

After extended exposure to dexamethasone, post-natal blastomere-like stem cells differentiated into more than 50 discrete cell types. The induced cell types exhibited characteristic morphological and phenotypic expression markers for spermatogonia, pluripotent epiblastic-like stem cells, ectodermal germ layer lineage stem cells, epidermal progenitor cells, epidermal cells, neuronal progenitor cells, dopaminergic neurons, pyramidal neurons, other types of neurons, astrocytes, oligodendrocytes, radial glial cells, ganglion cells, endodermal germ layer lineage stem cells, gastrointestinal epithelial cells, hepatic progenitor cells, hepatocytes, bile canalicular cells, oval cells, pancreatic progenitor cells, pancreatic ductal cells, pancreatic alpha-cells, pancreatic beta-cells, pancreatic delta-cells, three-dimensional pancreatic islets, mesodermal germ layer lineage stem cells, muscle progenitor cells, skeletal muscle, smooth muscle, cardiac muscle, adipogenic progenitor cells, white fat, brown fat, chondrogenic progenitor cells, hyaline cartilage, articular cartilage, growth plate cartilage, elastic cartilage, fibrocartilage, fibrogenic progenitor cells, tendon, ligament, scar tissue, dermis, osteogenic progenitor cells, cancellous bone, trabecular bone, woven bone, lamellar bone, osteoblasts, osteocytes, osteoclasts, endotheliogenic progenitor cells, endothelial cells, hematopoietic progenitor cells, erythrocytes, macrophages, B-cell lymphocytes, and T-cell lymphocytes.

Such induced unidirectional lineage-commitment process necessitates the use of either general induction agents or those that cause the cell to differentiate into specific lineages. It is contemplated that once blastomere-like stem cells are induced to commit to pluripotent epiblast-like stem cells, they have four options. The stem cells can (a) apoptose, (b) remain quiescent, (c) proliferate, or (d) differentiate into ectodermal, endodermal, and/or mesodermal germ layer lineage stem cells. Similarly, once pluripotent epiblastic-like stem cells are induced to commit to form ectodermal, endodermal, and/or mesodermal germ layer lineage stem cells, they have four options. The stem cells can (a) apoptose, (b) remain quiescent, (c) proliferate, or (d) differentiate into lineage-committed progenitor cells characteristic of specific tissue lineages. Once committed, they assume the characteristics of lineage-specific progenitor cells, which again can apoptose, remain quiescent, proliferate, or uni-directionally progress down their differentiation pathway, under the influence of specific agents. As committed progenitor cells, their ability to replicate is limited to approximately 50-70 cell doublings (human) or 8-10 cell doublings (rodent) before programmed cell senescence and cell death occurs.

Consequently, it should be recognized that human post-natal totipotent stem cells can be obtained in a relatively simple manner and expanded without differentiation over at least 10 generations, more typically over at least 50 generations, even more typically over at least 100 generations, and most typically over at least 200 generations. Indeed, previous experiments by the inventors have shown that the cells according to the inventive subject matter can undergo at least 100, more typically at least 200, even more typically at least 300 doublings in a serum-free defined propagation medium in the absence of differentiation inhibitors (see below). Therefore, it should be recognized that these cells do not spontaneously differentiate in serum-free defined propagation medium in the absence of differentiation inhibitors. Once sufficient quantities of BLSC are obtained (with or without expansion), they may be implanted into a human without teratoma formation, and will remain quiescent unless in the presence of damaged, necrotic, and/or inflamed tissue undergoing repair. Alternatively, contemplated BLSC may be expanded *in vitro* and then subjected to differentiation steps to thereby generate pluripotent stem cells (e.g., epiblast-like stem cells), germ layer lineage stem cells (e.g., those forming ectodermal cells, mesodermal cells, and endodermal cells), and/or progenitor cells (e.g., multipotent, tripotent, bipotent, and unipotent) in quantities that would otherwise be difficult, if not even impossible to obtain. Moreover, it should be recognized that such cells will be available for implantation into a donor with either an autogenic or allogenic match.

### Experiments

The following descriptions and protocols are provided to give exemplary guidance to a person to make and use various aspects of the inventive subject matter presented herein. However, it should be appreciated that numerous modifications can be made without departing from the spirit of the present disclosure. Further contemplations, considerations, and experimental details are provided in WO 01/21767, U.S. Pat. App. Nos. 2003/0161817, and 2004/0033214, all of which are incorporated by reference herein.

### Solutions, Media, and Supplies

Bleach Solution: 0.5% Sodium hypochlorite (undiluted Clorox).

Disinfectant: The disinfectant of choice is Amphyl solution: 0.5% (v/v) in deionized water. In a 20 L carboy add 100 ml of Amphyl (catalog # 21899-600, VWR International, Bristol, CT) and then add 20 L of deionized water. However, 70% ethanol or other disinfectants not harmful to the cells may be utilized.

70% (v/v) Ethanol: Dilute 95% ethanol to 70% (v/v) with double deionized water. In a 500 ml glass media bottle, mix 368.4 ml of 95% ethanol with 131.6 ml of double deionized water. Store solution at ambient temperature.

Sterile 5M sodium hydroxide: Weigh out 20 g of sodium hydroxide granules (catalog # S318, Fisher Scientific, Pittsburgh, PA) and add to a glass media bottle. Very slowly add 100 ml of double deionized water to the sodium hydroxide granules. Once the sodium hydroxide is dissolved, filter sterilize the solution through a 0.1 µm bottle top vacuum filter. Store the solution at ambient temperature.

Sterile 5M hydrochloric acid: Measure 58.3 ml of double deionized distilled water and place in a 100-ml glass media bottle. Measure 41.7 ml of 12 M HCl (catalog # 5619-02, VWR, JT5619-2, Bristol, CT) and very slowly add to water. Place cap on bottle and swirl gently to mix contents. Filter sterilize the solution through a 0.1 µm bottle top vacuum filter. Store the solution at ambient temperature.

0.4% Trypan Blue solution: Weigh out 0.2 g of Trypan blue (catalog # 11618, Eastman Kodak Company, Rochester, NY) and place in a sterile 100 ml glass media bottle. Under sterile conditions using a 25 ml pipette, add 50 ml of sterile Rinse buffer (catalog # MBC-ASB-REB-200-A001, Moraga Biotechnology Corp., Los Angeles, CA. Fax: 310-440-0437; Tel 310-440-0374) containing 1% (or 1 ml of the 100X) antibiotic-antimycotic solution (catalog # 15240-104, GIBCO), at pH 7.4. Swirl bottle gently to dissolve the trypan blue powder. Filter sterilize the trypan blue solution through a 0.2 µm bottle-top vacuum filter. Store this solution at ambient temperature.

Sterile Rinse Buffer with Ca⁺²/Mg⁺², pH 7.4: Under sterile conditions, take a fresh 500 ml bottle of sterile Rinse Buffer with Ca⁺²/Mg⁺² (catalog # MBC-ASB-REB-200-A001, Moraga Biotechnology Corp.), discard 5 ml to bleach, and then add 5 ml of the 100X antibiotic-antimycotic solution (catalog # 15240-104, GIBCO), for a final concentration of 1X. Invert the bottle a few times to mix the solution, and bring the pH to 7.4 using sterile 5M sodium hydroxide. Store the solution at 4°C.

Sterile Release Buffer without Ca⁺²/Mg⁺², pH 7.4: Under sterile conditions, take a fresh 500 ml bottle of sterile Release Buffer without Ca⁺²/Mg⁺² (catalog # MBC-ASB-REB-200-A002, Moraga Biotechnology Corp.), discard 5 ml to bleach, and then add 5 ml of the 100X antibiotic-antimycotic solution (catalog # 15240-104, GIBCO), for a final concentration of 1X. Invert the bottle a few times to mix the solution, and bring the pH to 7.4 using sterile 5M sodium hydroxide. Store the solution at 4°C.

Sterile SFD-BLSC Rinse Buffer, Ca⁺²/Mg⁺², pH 7.4: Under sterile conditions, take a fresh 500 ml bottle of sterile serum-free-defined (SFD)-BLSC Rinse Buffer with Ca⁺²/Mg⁺² (catalog # MBC-ASB-REB-100-A001, Moraga Biotechnology Corp.), discard 5 ml to bleach, and then add 5 ml of the 100X antibiotic-antimycotic solution (catalog # 15240-104, GIBCO), for a final concentration of 1X. Invert the bottle a few times to mix the solution, and bring the pH to 7.4 using sterile 5M sodium hydroxide. Store the solution at 4°C.

Sterile SFD-BLSC Release Buffer without Ca⁺²/Mg⁺², pH 7.4: Under sterile conditions, take a fresh 500 ml bottle of sterile serum-free defined (SFD) BLSC Release Buffer without Ca⁺²/Mg⁺² (catalog # MBC-ASB-REB-100-A002, Moraga Biotechnology Corp.) and discard 5.0 ml to bleach. Add 5 ml of the 100X antibiotic-antimycotic solution (catalog # 15240-104, GIBCO) to the glass bottle (final concentration of 1X). Swirl to mix contents. Adjust the pH of the solution to 7.4 with 5 M sodium hydroxide and/or 5 M hydrochloric acid. Store this solution at 4°C.

Dexamethasone solution, pH 7.4: This must be made up in absolute ethanol (EtOH) because it is not soluble in water or media. Weigh out 0.039 g of Dexamethasone (Dex, catalog # D-1756, Sigma) and add to 10 ml of absolute EtOH. This will make a 1 x 10⁻² M stock solution. Store this solution at -20°C. This is the most concentrated solution of Dex that can be made with complete solubility. Add 1 ml of the stock Dex solution made above to 9-ml Opti-MEM I medium with Glutamax. Aliquot 9 ml of this solution as 500µl quantities in 2 ml cryovials and store at -20°C. Label these tubes as 1 x 10⁻⁶M Dex. Take the remaining 1 ml of 10⁻⁶M Dex and add to 9 ml of Opti-MEM I medium with Glutamax. Aliquot 9 ml and reserve 1 ml as before. Label these tubes as 1 x 10⁻⁷M Dex. Take the remaining 1 ml of 10⁻⁷M Dex and add to 9 ml of Opti-MEM I medium with Glutamax. Aliquot 9 ml and reserve 1 ml as before. Label these tubes as 1 x 10⁻⁸M Dex. Take the remaining 1 ml of 10⁻⁸M Dex and add to 9 ml of Opti-MEM I medium with Glutamax. Aliquot 9 ml and reserve 1 ml as before. Label these tubes as 1 x 10⁻⁹M Dex. Take the remaining 1 ml of 10⁻⁹M Dex and add to 9 ml of Opti-MEM I medium with Glutamax. Aliquot all 10 ml. Label these tubes as 1 x 10⁻¹⁰M Dex. These aliquots will bring 500 ml of media to the concentration of Dex labeled on the tube. Store the cryovials at -20°C.

Insulin solution, pH 7.4: Weight out 100 mg of Insulin (catalog #I-5500, Sigma) and add to a 15 ml centrifuge tube. Under sterile conditions, add 5.0 ml of Opti-MEM I media with Glutamax to the centrifuge tube. Invert the centrifuge tube to dissolve the insulin. Filter sterilize twice using a 0.2µm syringe filter, into a 15 ml centrifuge tube first and then a 50 ml centrifuge tube the second time. Measure volume using a 5 ml pipet. Add enough Opti-MEM I media with Glutamax to bring the volume up to 15 ml. The final concentration will be approximately 1mg/500µl. Aliquot this solution into 1-ml cryovials, at 500 µl each. Store the cryovials at -20°C. One aliquot will bring 500 ml of media up to the final concentration of 2 µg/ml insulin.

Sterile Serum-Free Defined BLSC Media Supplements, pH 7.4: Under sterile conditions remove 7.975 ml from 500-ml bottle of sterile tissue culture medium of choice (e.g., EMEM, RPMI, Opti-MEM, or etc.) and discard to bleach. Add 7.975-ml aliquot of SFD-BLSC Media Supplements (catalog # MBC-ASB-MED-100-A001, Moraga Biotechnology Corp.) and swirl the bottle gently to mix contents. Remove 5.0 ml of solution and discard to bleach. Add 5 ml Antibiotic-Antimycotic solution. Swirl the bottle gently to mix contents and pH to 7.4. Store at 4°C.

Serum-Free Defined BLSC Basal Medium, pH 7.4: Under sterile conditions remove 5.0 ml from 500 ml bottle of Serum-Free Defined BLSC Basal Medium (catalog # MBC-ASB-MED-100-A002, Moraga Biotechnology Corp.) and discard to bleach. Add 5 ml Antibiotic-Antimycotic solution. Swirl the bottle gently to mix contents and pH to 7.4. Store at 4°C.

Propagation Supplement, pH 7.4: Under sterile conditions remove 6.0 ml from 500 ml bottle of medium supplemented with Serum-Free Defined BLSC Media Supplements (catalog # MBC-ASB-MED-100-A001, Moraga Biotechnology Corp.) and discard to bleach. Add 1.0 ml of Propagation Supplement (catalog # MBC-ASB-MED-100-A003, Moraga Biotechnology Corp.) and 5 ml of Antibiotic-Antimycotic solution. Swirl the bottle gently to mix contents and pH to 7.4. Store at 4°C.

Serum-Free Defined BLSC Propagation medium, pH 7.4: Under sterile conditions remove 5.0 ml from 500 ml bottle of Serum-Free Defined BLSC Propagation medium (catalog # MBC-ASB-MED-100-A006, Moraga Biotechnology Corp.) and discard to bleach. Add 5 ml Antibiotic-Antimycotic solution. Swirl the bottle gently to mix contents and pH to 7.4. Store at 4°C.

Serum-Free Defined BLSC Transport medium, pH 7.4: Under sterile conditions remove 15.0 ml from 500 ml bottle of Serum-Free Defined BLSC Transport medium (catalog # MBC-ASB-MED-100-A004, Moraga Biotechnology Corp.) and discard to bleach. Add 15 ml Antibiotic-Antimycotic solution. Swirl the bottle gently to mix contents and pH to 7.4. Store at 4°C.

Serum-Free Defined BLSC Cryopreservation medium, pH 7.4: Under sterile conditions, take a fresh 100 ml bottle of Serum-Free Defined BLSC Cryopreservation Medium, pH 7.4 (catalog # MBC-ASB-MED-100-A005, Moraga Biotechnology Corp.). Remove 1.0 ml of medium and discard to bleach. Add 1 ml Antibiotic-Antimycotic solution. Swirl the bottle gently to mix contents and pH to 7.4. Store at 4°C.

General Induction medium, pH 7.4: Serum-Free Defined BLSC Propagation Medium, pH 7.4, containing 10⁻⁸ M dexamethasone, 2 µg/ml insulin, 5% SS9, and 10% SS12. Under sterile conditions, take a fresh 500 ml bottle of SFD-BLSC Propagation medium (catalog # MBC-ASB-MED-100-A006, Moraga Biotechnology Corp.) remove 83 ml of medium and place into a sterile 100-ml bottle. Add 500 µl aliquot of insulin, 500 µl aliquot of dexamethasone, 5 ml of SS9 (catalog # H7889, Sigma), and 10 ml of SS12 (catalog # FB-01, Omega Scientific, Tarzana, CA). Swirl the bottle gently to mix solutions, pH to 7.4 and store at 4°C.

Ectodermal Induction medium, pH 7.4: Serum-Free Defined BLSC Propagation medium, containing 10⁻⁸ M dexamethasone, 2 µg/ml insulin, and 15% SS12, pH 7.4. Under sterile conditions, take a fresh 500 ml bottle of Serum-Free Defined BLSC Propagation medium, pH 7.4, and remove 83 ml of medium and place into a sterile 100-ml bottle. Add 500 µl aliquot of insulin, 500 µl aliquot of dexamethasone, and 15 ml of SS12 (catalog # FB-01, Omega Scientific, Tarzana, CA). Swirl the bottle gently to mix solutions and store at 4°C.

Mesodermal Induction medium, pH 7.4: Serum-Free Defined BLSC Propagation medium, containing 10⁻⁸ M dexamethasone, 2 µg/ml insulin, and 10% SS9, pH 7.4. Under sterile conditions, take a fresh 500 ml bottle of Serum-Free Defined BLSC Propagation medium, pH 7.4, and remove 83 ml of medium and place into a sterile 100-ml bottle. Add 500 µl aliquot of insulin, 500 µl aliquot of dexamethasone, and 10 ml of SS9 (catalog # H7889, Sigma). Swirl the bottle gently to mix solutions and store at 4°C.

Endodermal Induction medium, pH 7.2: Serum-Free Defined BLSC Propagation medium, containing 10⁻⁸ M dexamethasone, 2 µg/ml insulin, and 15% SS12, pH 7.4. Under sterile conditions, take a fresh 500 ml bottle of Serum-Free Defined BLSC Propagation medium, pH 7.4, and remove 83 ml of medium and place into a sterile 100-ml bottle. Add 500 µl aliquot of insulin, 500 µl aliquot of dexamethasone, and 10 ml of SS12 (catalog # FB-01, Omega Scientific, Tarzana, CA). pH to 7.2 with 6 M HCl. Swirl the bottle gently to mix solutions and store at 4°C.

SFD-Tissue Release Solution, pH 7.4: SFD-Tissue Release Solution (catalog # MBC-ASB-RED-100-A003, Moraga Biotechnology Corp.), store the tubes at -20°C until needed. Just before use, thaw, remove 1% solution and discard to bleach. Add 1% antibiotic-antimycotic solution and pH to 7.4.

SFD-Cell Release/Activation solution, pH 7.4: Under sterile conditions, take a fresh 500 ml bottle of SFD-Cell Release/Activation Solution (catalog # MBC-ASB-RED-100-A004, Moraga Biotechnology Corp.), remove 5.0 ml of solution and discard to bleach. Add 5 ml Antibiotic-Antimycotic solution. Swirl the bottle gently to mix contents and pH to 7.4. Store at 4°C.

SFD-Cell Release/Activation Inhibitor Solution, pH 7.4: Under sterile conditions, take a fresh 500 ml bottle of SFD-Cell Release/Activation Solution Inhibitor (catalog # MBC-ASB-RED-100-A005, Moraga Biotechnology Corp.), remove 5.0 ml of solution and discard to bleach. Add 5 ml Antibiotic-Antimycotic solution. Swirl the bottle gently to mix contents and pH to 7.4. Store at 4°C.

SFD-BLSC-MACS buffer, pH 7.2: Under sterile conditions, take a fresh 500 ml bottle of SFD-BLSC-MACS buffer (catalog # MBC-ASB-RED-100-A006, Moraga Biotechnology Corp.), remove 5.0 ml of solution and discard to bleach. Add 5 ml Antibiotic-Antimycotic solution. Swirl the bottle gently to mix contents and pH to 7.2. Store at 4 °C.

Adult Stem Cell Coated culture vessels: 75 cm² flasks (catalog # MBC-ASB-MSD-900-A006, Moraga Biotechnology Corp.), 25 cm² flasks (catalog # MBC-ASB-MSD-900-A007, Moraga Biotechnology Corp.), 6-well plates (catalog # MBC-ASB-MSD-900-A008, Moraga Biotechnology Corp.), 24-well plates (catalog # MBC-ASB-MSD-900-A009, Moraga Biotechnology Corp.), 48-well plates (catalog # MBC-ASB-MSD-900-A010, Moraga Biotechnology Corp.), and 96-well plates (catalog # MBC-ASB-MSD-900-A011, Moraga Biotechnology Corp.).

### Methods

### Tissue Harvest

Preferably, the isolated tissue comprises connective tissue as the source for totipotent blastomeric-like stem cells. The connective tissue may be associated with various organs and tissues. Here, tissue was harvested from the hind limb of a rat. However, it should be recognized that the tissue may be from any other mammal, and especially from a human (e.g., using muscle biopsy techniques well known in the art).

Put on gloves. Soak wipes with the disinfectant solution. Wipe your gloved hands with the wipes that have been soaked with disinfectant. Weigh a (non-human) animal, and calculate how much anesthetic agent will be required to anesthetize the animal. Use the appropriate anesthetic agent per 1 kg of body weight. Draw up the appropriate amount of anesthetic agent in a sterile syringe fitted with a 26 gauge needle. Disinfect the injection site with 70% (v/v) ethanol and allow to dry. Make an intraperitoneal injection through the abdominal wall of the rat. Once the rat is unconscious, disinfect the hind limbs and abdomen with 70% (v/v) ethanol, and allow these areas to dry by evaporation. Shave the hair from the abdomen and hind limbs using an Oster^{™} animal shears fitted with a #40 blade. Disinfect the shaved regions using a cotton ball soaked with Betadine. Allow the skin to dry by evaporation. Place a sterile #15 blade on a sterile #3 scalpel handle. Make an incision from the xiphoid process to the pubic symphysis. Euthanize the animal by cutting the diaphragm using dissecting scissors (4.5 inch). Immediately following euthanization, make two incisions using a sterile #15 scalpel blade attached to a #3 scalpel handle. Make one incision along the medial surface and one incision along the lateral surface of the thigh and leg. These incisions will aid in the removal of the skin. Remove the skin from each hind limb using the tissue forceps and a scalpel. Cut the quadriceps femoris tendon and the proximal origins of the anterior thigh muscles with the scalpel. Using the tissue forceps, strip the anterior thigh muscles away from the femur.

Place the relatively intact anterior thigh muscles into a 50 ml centrifuge tube containing 25 ml of cold (4°C) Serum-Free Defined (SFD-) BLSC Transport medium, pH 7.4. Remove the posterior thigh muscles using a scalpel to cut through the proximal and distal attachments of these muscles. Place the posterior thigh muscles in another 50 ml centrifuge tube containing 25 ml of cold (4°C) SFD-BLSC Transport medium, pH 7.4. Place both 50 ml centrifuge tubes on ice until transport. Repeat this procedure on the other hind limb. Transport the tissue on ice to the tissue culture lab. Store the tissue in transport medium in the refrigerator at 4° C for a maximum of five (5) days.

### Isolating Cells From Connective Tissue

Put on gloves. Soak wipes with the disinfectant solution. Wipe your gloved hands with wipes that have been soaked in disinfectant. Wipe all the inside surfaces of the class II biosafety cabinet with wipes that have been soaked in disinfectant. Allow the cabinet to dry by evaporation. Wipe the outside of the counter top with wipes that have been soaked in disinfectant. Allow the countertop to dry by evaporation. Wipe the outside surfaces of all supplies with a wipes that has been soaked in disinfectant before placing the supplies in the class II biosafety cabinet. Remove the 50 ml tubes that contain the tissue in transport solution from the refrigerator. Wipe the outside of the 50 ml tubes with wipes soaked in disinfectant. Place the 50 ml tubes containing the tissue in the class II biosafety cabinet.

Pipet 10 ml of fresh sterile SFD-BLSC Transport medium into each of four sterile 100 mm glass Petri dishes (one dish for each 50 ml tube of tissue). Use the sterile forceps to transfer each set of muscle tissues into a separate sterile 100 mm glass Petri dish. Examine the muscle tissue using a dissecting microscope. Using the tissue forceps and dissecting scissors, remove and discard tendons, major blood vessels (such as the femoral artery and vein, and the profunda femoris artery), and major nerves (such as the sciatic nerve, tibial nerve, and common fibular nerve). The remaining tissue should consist predominantly of muscle myofibers and the adherent connective tissue coverings (i.e., epimysium, perimysium, and endomysium). Smaller associated nerve branches and vascular tissues will also remain. Use the dissecting scissors to cut the muscle tissue into 1 cm² pieces. Place the pieces of muscle tissue and the associated connective tissues from a particular muscle group (such as the anterior thigh muscle of the right lower limb) into a sterile 60 mm glass Petri dish containing 10 ml of proliferation medium. Carefully mince the muscle tissue using sterile dissecting scissors and very fine pointed sterile forceps. Continue mincing the tissue until it has the consistency of orange marmalade. Take aliquots of approximately 5 ml of the minced tissue and place them in sterile 50 ml centrifuge tubes.

Centrifuge the 50 ml centrifuge tubes containing the minced tissue at 2000 x g for 5 minutes at ambient temperature. Discard the supernatant by placing it in the bleach solution. Estimate the volume of each tissue pellet. Resuspend the tissue pellets by raking the centrifuge tubes across an 80-well microtube holder, 12-15 times. Add 7 pellet volumes of the Serum-Free Defined BLSC Propagation medium and 2 pellet volumes of the SFD-Tissue Release Solution to each tissue suspension. Vortex each centrifuge tube. Cut a single square of Parafilm and wipe each side with wipes soaked in disinfectant. Fold the Parafilm in half and stretch it. Wrap the double layer of Parafilm around the interface of the cap and the tube of each 50 ml centrifuge tube and seal it. Place the sealed 50 ml centrifuge tubes into a (Gladware^{™}) container. Place the lid on the (Gladware) container. Cut a strip of 10 single squares of Parafilm and wipe each side with wipes soaked in disinfectant. Fold the Parafilm in half, stretch it, and wrap it around the interface of the (Gladware) lid and container to seal it. Place the sealed (Gladware) container in a 37°C shaking water bath and set the shaking speed to low medium. Allow the tissue/enzyme mixture to shake at 37°C until the tissue is digested. The tissue is digested when no visible tissue clumps remain and the tissue is liquefied. Once the tissue has been completely digested, remove the container from the shaking water bath. Remove the tubes from the container.

Centrifuge the 50 ml centrifuge tubes containing the digested tissue at 2000 x g for 5 minutes at ambient temperature. Discard the supernatant by placing it in the bleach. Be sure to leave a small amount of the supernatant, about equal the volume of the cell pellet, in the tube. This can be accomplished using one of two methods. The first method involves pouring off the supernatant into the bleach solution. The second method involves aspirating the supernatant using a Pasteur pipette attached to vacuum aspirator. Be careful not to dislodge the cell pellet with the Pasteur pipette. Resuspend the cell pellet in the residual supernatant by raking the centrifuge tube longitudinally across an 80-well microtube holder. Repeat this procedure 12-15 times. Reconstitute the cell pellet in 20 ml of proliferation medium.

### Sieving the Cell Suspension through Nitex Filters

Set up a sterile 90 µm Nitex filter apparatus on top of a sterile 100 ml glass media bottle. Pre-wet the 90 µm Nitex filter with Serum-Free Defined (SFD-) BLSC Propagation medium. To accomplish this step, place 10 ml of SFD-BLSC Propagation medium into the barrel of the 50 ml syringe. Allow the medium to percolate by gravity through the filter to saturate the membrane. The membrane is saturated when a few drops of medium appear in the bottle. If drops do not appear in the bottle, repeat the wetting procedure until drops appear within the bottle. Place the cell suspension into the barrel of the 50 cc syringe and allow it to flow by gravity through the filter. Once the cell suspension has completely passed through the filter, wash the 90 µm filter apparatus with 10 ml of the fresh SFD-BLSC Propagation medium. Remove the 100 ml media bottle from the 90 µm filter apparatus and cap the bottle. Remove the 90 µm Nitex filter from the unit and place it into a 50 ml centrifuge tube containing 10 ml of SFD-BLSC Propagation medium. Vortex the centrifuge tube on medium speed for 3 pulses at about 1 second each to release the cells. Place the cell suspension into an Adult Stem Cell Coated 75 cm² flask. Label the flask using a permanent marker. Rock the tissue culture flask from side to side to disperse the cell suspension. Place the flask into a humidified incubator that uses an environment of 95% air/5% carbon dioxide and is set at 37° C.

Set up a 20 µm Nitex filter apparatus on top of a clean sterile 100 ml glass media bottle. Pre-wet the Nitex filter with SFD-BLSC Propagation medium, as described in above. Take the cell suspension that has been filtered through the 90 µm filter and place it into the 50 cc syringe tube for the 20 µm Nitex filter. Allow the suspension to pass by gravity through the filter. Wash the 20 µm filter apparatus with 10 ml of fresh proliferation medium. Remove the 100 ml media bottle from the 20 µm filter apparatus and cap it. Remove the 20 µm filter from the unit and place it into a 50 ml centrifuge tube containing 10 ml of SFD-BLSC Propagation medium. Vortex the centrifuge tube at medium speed for 3 pulses of 1 second each to release the cells. Place the cell suspension into an Adult Stem Cell Coated 75 cm² flask. Label the flask using a permanent marker. Rock the tissue culture flask from side to side to disperse the cell suspension. Place the flask into a humidified incubator that uses an environment of 95% air/5% carbon dioxide and is set at 37° C. Divide the sieved cell suspension into equal volumes and place them in sterile 15 ml centrifuge tubes. Centrifuge the sieved cell suspension at 2000 x g for 5 minutes at ambient temperature. After centrifugation, discard the supernatant from all centrifuge tubes by placing it in bleach solution. Be sure to leave a small amount of the supernatant, about equal to the volume of the cell pellet. Resuspend the cell pellets by raking the centrifuge tubes across an 80-well microtube holder. Repeat 12-15 times. Using a 5 ml pipet and starting with 5 ml of SFD-BLSC Propagation medium, wash and triturate each 15 ml centrifuge tube in sequence. Combine the cell suspensions. Place the combined cell suspension in a 15 ml centrifuge tube. Using a 10 ml pipet and starting with 5 ml of proliferation medium, rewash and triturate each 15 ml centrifuge tube in sequence. Combine the rewashes. Add the rewash to the cell suspension in the 15 ml tube. Triturate the cell suspension gently, 10-12 times.

### Counting the Cells

Measure and record the total volume of the combined cell suspension. Remove 0.1 ml of the cell suspension, and place it into a 1.7 ml microcentrifuge tube. Add 0.1 ml of 0.4% trypan blue solution to the 0.1 ml of the cell suspension and triturate 6-8 times gently to mix. Remove 100 µl of the trypan blue/cell mixture, load the hemocytometer, and examine under a light microscope with a 10x objective. Determine the number of BLSC versus non-BLSC by trypan blue inclusion/exclusion. BLSC (< 1 µm in size) do not exclude trypan blue and appear blue-stained. In contrast, viable epiblast-like stem cells (6-8 µm in size), germ layer lineage stem cells (10-20 µm in size), progenitor cells (variable, but > 5 µm in size), and differentiated cells (variable, but > 5 µm in size), exclude the trypan blue dye and appear to be clear. Calculate the total number of cells per ml of cell suspension by counting the total number of stained and unstained cells present in a volume of the cell suspension. This is accomplished by counting all the cells present in the nine large grids on the hemocytometer. Dead cells are blue in color and > 5 mm in size. Next calculate the total number of viable epiblast-like stem cells, germ layer lineage stem cells, progenitor cells, and differentiated cells per ml of cell suspension by counting the total number of clear/refractile cells present in a volume of the cell suspension. Next calculate the total number of dead cells by counting the number of trypan-blue stained cells of similar size to the clear-appearing cells. To calculate the number of BLSC, subtract the cells greater than 5 µm (viable cells + dead cells) from the total number of cells using the formula: [(total number of cells) - (cells > 5 µm viable cells + dead cells) = BLSC]. Calculate the average numbers of cells (BLSCs and combined other cell types) for each large grid. Calculate the number of BLSCs and combined other cell types per ml of cell suspension. Determine the total number of cells.

### Plating the Cells

Use initial cell densities of 0.5 to 2.0 x 10⁶ cells per 5 ml of SFD-BLSC Propagation medium for Adult Stem Cell Coated 25 cm² flasks and 2.0 to 4.0 x 10⁶ cells per 10 ml of SFD-BLSC Propagation medium for Adult Stem Cell Coated 75 cm² flasks. To plate the cells, first determine the volume of the cell suspension needed to yield the required number of cells for plating. Next subtract the volume of the cell suspension from the flask volume (5 ml for 25 cm² flasks and 10 ml for 75 cm² flasks), using the formula: [(flask volume - cell suspension volume) = residual volume]. Pre-wet the flask surface to disperse surface tension with the residual volume of medium. Rock the flask back and forth and side to side so that the surface of the flask is completely covered. Add the cell suspension volume to the flask. Evenly distribute the cells across the surface of the flask by rocking the flask back and forth and side to side. Label the flasks using a permanent marker. Place the flask(s) into a humidified incubator that uses an environment of 95% air/5% carbon dioxide and is set at 37° C.

### Cultivating the BLSCs in Suspension

After initial plating, these cells must be observed daily until after the first passage and cared for appropriately depending on visual observations of the cultures. For example, the epiblast-like stem cells, germ layer lineage stem cells, and progenitor cells will attach to the Adult Stem Cell Coated flask surface within 18 to 24 hours after plating; in contrast, blastomere-like stem cells remain in suspension. Therefore, in the initial plating medium after attachment there will be many types of floating cells, blastomere-like stem cells, damaged cells, lysed cells, cell debris, intracellular enzymes, intracellular organelles, etc. The cellular debris must be removed from the culture medium to ensure the subsequent viability of the blastomere-like stem cells.

Allow the epiblast-like stem cells, germ layer lineage stem cells, and progenitor cells a minimum of 18-24 hours to attach to the surface of the flask. Put on gloves. Soak wipes with disinfectant solution. Wipe the gloved hands with wipes soaked in disinfectant. Wipe all the inside surfaces of the class II biosafety cabinet with wipes that have been soaked with disinfectant. Allow the surfaces to dry by evaporation. Wipe the outside counter top with wipes that have been soaked with disinfectant. Allow the surfaces to dry by evaporation. Wipe the outside surfaces of all supplies with wipes that have been soaked with disinfectant before placing the supplies into the class II biosafety cabinet. Twenty-four hours after cell plating the original medium is removed to 15-ml polypropylene centrifuge tubes. This can be accomplished either by pouring medium into the tubes or pipetting medium into the tubes. Tubes are spun at 2000 x g for 5 minutes to pellet the BLSCs. The cell debris, intracellular enzymes, intracellular organelles, etc., remain in suspension. Pour the supernatant into the bleach solution. Be sure to leave a small amount of the supernatant, about equal to the volume of the cell pellet. Wash the cell pellet an additional two times to remove residual cell debris. This is accomplished by resuspending the cell pellet by raking the centrifuge tube across an 80-well microtube holder. Repeat 12-15 times. Add sufficient volume of SFD-BLSC Propagation medium to bring tube volume to 14 ml. Tubes are spun at 2000 x g for 5 minutes to pellet the BLSCs. Pour the supernatant into the bleach solution. Be sure to leave a small amount of the supernatant, about equal to the volume of the cell pellet. Repeat procedure.

Count BLSCs, as above, to determine inoculation densities for cell growth. Use initial cell inoculation densities of 0.5 x 10⁶ cells per 5 ml of SFD-BLSC Propagation medium for Adult Stem Cell Coated 25 cm² flasks and 1.0 x 10⁶ cells per 10 ml of SFD-BLSC Propagation medium for Adult Stem Cell Coated 75 cm² flasks.

Feed the culture with fresh SFD-BLSC Propagation medium, and return it to the incubator. Add medium to the flasks every 24-48 hours, depending on the percentage of cells within the flask(s). [For example, when the approximate percentage of the cells in the flask is less than 50%, feed the culture(s) with 5 ml (per 25 cm² flask) or 10 ml (per 75 cm² flask) of medium. When the approximate percentage of the cells in the flask is 60-70%, feed the culture(s) with 10-15 ml (per 25 cm² flask) or 20-30 ml (per 75 cm² flask) of medium. Once the approximate percentage of the cells in the flask is greater than 75%, harvest the cells from the flask and divide into new flasks with a starting inoculation density of 0.5 x 10⁶ cells per 5 ml of SFD-BLSC Propagation medium for 25 cm² flasks or 1.0 x 10⁶ cells per 10 ml of SFD-BLSC Propagation medium for 75 cm² flasks.

### Harvest Cells from the Flask

Totipotent stem cells grow in suspension in SFD-BLSC Propagation medium. Therefore, they will continue to proliferate as long as they are maintained with proliferation medium, e.g., SFD-BLSC Propagation medium. Once the approximate percentage of the cells in the flask is greater than 75%, harvest the cells from the flask. Put on gloves. Soak wipes with disinfectant solution. Wipe gloved hands with wipes that have been soaked in disinfectant. Wipe all inside surfaces of the class II biosafety cabinet with wipes that have been soaked with disinfectant solution. Allow them to dry by evaporation. Wipe the outside of the counter top with wipes that have been soaked with disinfectant solution. Allow the counter top to dry by evaporation. Wipe the outside surfaces of all supplies with wipes that have been soaked with disinfectant solution before placing the supplies into a Class II Biosafety cabinet.

Prepare sterile 15-ml polypropylene centrifuge tubes by wiping outside with disinfectant agent and placing in sterile Class II Biosafety cabinet. Under sterile conditions remove 14 ml cell suspension from tissue culture flask and place into each tube. This can be accomplished by either pouring or pipetting the cell suspension from the flask into the tube(s). Centrifuge the 15-ml polypropylene centrifuge tubes at 2000 x g for 5 minutes at ambient temperature. After centrifugation, discard the supernatant from the centrifuge tube by placing it in the bleach solution. Be sure to leave a small amount of the supernatant, about equal the volume of the cell pellet. Resuspend the cell pellet by raking the centrifuge tubes across an 80-well microtube holder. Repeat this process 12-15 times.

Use a 5 ml pipette to wash and triturate each 15 ml centrifuge tube in sequence. Use 1-5 ml of SFD-BLSC Propagation medium in this process. The volume to be used will depend upon the volume of the cell suspension to be resuspended. Place the combined cell suspension in a 15 ml centrifuge tube. Count the cells as outlined above.

Divide cells into new flasks or cryopreserve cells. Inoculate new cultures with a starting density of 0.5 x 10⁶ cells per 5 ml of SFD-BLSC Propagation medium for Adult Stem Cell Coated 25 cm² flasks or 1.0 x 10⁶ cells per 10 ml of SFD-BLSC Propagation medium for Adult Stem Cell Coated 75 cm² flasks. Process cultures as described above for propagation.

### Release of Adherent Cells

Epiblast-like stem cells, germ layer lineage stem cells, progenitor cells, and differentiated cells adhere to flasks coated in gelatin. If any or all of these cell types are utilized for subsequent characterization assays, or otherwise utilized, they must be removed from their respective Adult Stem Cell Coated propagation flasks. This is accomplished as follow.

Under sterile conditions, add 2 ml of SFD-Cell Release/Activation Inhibitor Solution, pH 7.4, to a 15 ml centrifuge tube. Repeat this step for each flask of cells that will be released with the SFD-Cell Release/Activation Solution. Discard the medium from the culture flask by placing it into the bleach solution. Wash the culture flask with Sterile Rinse Buffer with Ca⁺²/Mg⁺², pH 7.4: 13 ml for the 25 cm² flask and 35 ml for the 75 cm² flask. Wait a minimum of 5 minutes and then discard the wash solution by placing it in the bleach solution. Repeat this wash procedure one more time.

Wash the culture flask with Sterile Release Buffer without Ca⁺²/Mg⁺², pH 7.4: 10 ml for the 25 cm² flask and 25 ml for the 75 cm² flask. Wait a minimum of 5 minutes and discard the wash solution by placing it in the bleach solution. Add 4 ml of SFD-Cell Release/Activation solution, pH 7.4, to the flask to release the cells from the surface of the flask. The cells will lift off in 2-3 minutes. Gently rock the culture flask side to side to enhance the release process. Once the cells have been released from the flask surface, use a 5 ml pipette to triturate the cells into suspension. Wash the flask surface with the cell suspension. Remove the cell suspension from the flask and place it into a 15 ml tube containing the heat inactivated serum. Visually inspect the flasks to make sure that the cells have been released from the surface of the flask. Wash the flasks with 2 ml of SFD-BLSC Propagation medium to ensure that more than 99% of the cells have been released from the surface of the flask. Add the wash solutions to 15 ml centrifuge tubes that contain the SFD-Cell Release/Activation-Inhibitor Solution. Fill the 15 ml centrifuge tube containing the cell suspension, trypsin, and SFD-Cell Release/Activation Solution to the 14 ml mark with SFD-BLSC Propagation medium. Gently invert the tube twice to mix the contents. Centrifuge the tube at 2000 x g for 5 minutes at ambient temperature. After centrifugation, discard the supernatant from the centrifuge tube by placing it in the bleach solution. Be sure to leave a small amount of the supernatant, about equal the volume of the cell pellet. Resuspend the cell pellet by raking the centrifuge tubes across an 80-well microtube holder. Repeat this process 12-15 times.

Use a 5 ml pipette to wash and triturate each 15 ml centrifuge tube in sequence. Use 1-5 ml of SFD-BLSC Propagation medium in this process. The volume to be used will depend upon the volume of the cell suspension to be resuspended. Place the combined cell suspension in a 15 ml centrifuge tube. Count the cells as outlined above.

### Cryopreservation of Totipotent Stem Cells

Adult totipotent blastomeric-like stem cells are to be cryopreserved by slow freezing and storage at -50°C to -100°C. Put on gloves. Soak the wipes with the disinfectant solution. Wipe gloved hands with wipes that have been soaked in disinfectant solution. Wipe all inside surfaces of a class II biosafety cabinet with wipes that have been soaked in disinfectant solution. Allow them to dry by evaporation. Wipe the outside counter top with wipes that have been soaked in disinfectant solution. Allow the counter top to dry by evaporation. Wipe the outside surfaces of all supplies with wipes that have been soaked in disinfectant solution before placing the supplies into a class II biosafety cabinet.

Determine the number of cryovials to be used, based on the cell counts. The optimum range of final cell density for cryopreservation is 1 - 2 x 10⁶ cells per ml; therefore cells should be diluted to 2 - 4 x 10⁶ cells per ml for cryopreservation. Label the cryovials. Wipe the outside of the vials with wipes that have been soaked with disinfectant solution. Place the cryovials on an 80-well microtube holder. Pipet 0.5 ml of cellular suspension into each cryovial. Add 0.5 ml of SFD-BLSC Cryopreservation medium to each tube. Tighten the caps of the cryovials. Gently invert the cryovials twice to mix their contents. Gently place cryovials into a freezing chamber containing 100% isopropyl alcohol. Place the freezing chamber into a -50 °C to -100°C freezer. Allow freezing and storage for a minimum of 12 hours before thawing and plating the cells.

### Thawing the Frozen Cells for Plating

Put on gloves. Soak wipes with disinfectant solution. Wipe gloved hands with wipes that have been soaked in disinfectant solution. Wipe all inside surfaces of a class II biosafety cabinet with wipes that have been soaked in disinfectant solution. Allow the surfaces to dry by evaporation. Wipe the outside counter top with wipes that have been soaked in disinfectant solution. Allow the surfaces to dry by evaporation. Wipe the outside surfaces of all supplies with wipes that have been soaked in disinfectant solution before placing the supplies into a class II biosafety cabinet. Determine the number of cryovials of frozen cells to be used, based on the composition of the cellular constituents and the cell counts. Use one 15 ml centrifuge tube per cryovial.

Pipet 13 ml of SFD-BLSC Propagation medium at ambient temperature into each 15 ml centrifuge tube. Remove the cryovials from the freezer. Flash-thaw the frozen cellular suspension in the cryovials. This can be accomplished by numerous methods, including placing cryovials in an ambient temperature water bath until just thawed or place cryovial in gloved hand and allow body heat to just thaw cellular suspension. (In any procedure do not let temperature of cell suspension rise above ambient temperature.) Remove the thawed cellular suspension gently using a 1 ml pipette. Add the cellular suspension drop-wise to a 15 ml tube containing 13 ml of SFD-BLSC Propagation medium. Tighten the screw cap. Gently invert the tube twice to mix its contents. Centrifuge the tube at 2000 x g for 5 minutes at ambient temperature. After centrifugation, discard the supernatant from the centrifuge tube by placing it in the bleach solution. Be sure to leave a small amount of the supernatant, about equal to the volume of the cellular pellet. Resuspend the cellular pellet by raking the centrifuge tube across an 80-well microtube holder. Repeat this process 12-15 times.

Using a 5 ml pipette and starting with 2 ml of SFD-BLSC Propagation medium, wash and triturate each 15 ml centrifuge tube in sequence, and combine the cellular suspensions. Place combined cell suspension into a 15 ml centrifuge tube. Count the cells, plate and cultivate as outlined above.

### Segregation of Totipotent Blastomere-Like Stem Cells Utilizing Cell Surface Epitopes

Removal of germ layer lineage stem cells: Adult stem cells can be segregated based on their unique profiles of cell surface epitopes. At least three different techniques can be utilized, i.e., flow cytometry, magnetic bead sorting, and panning. The specific description given below is representative of magnetic bead sorting.

Put on gloves. Soak wipes with disinfectant solution. Wipe gloved hands with wipes that have been soaked in disinfectant solution. Wipe all the inside surfaces of a class II biosafety cabinet with wipes that have been soaked in disinfectant solution. Allow the surfaces to dry by evaporation. Wipe the outside counter top with wipes that have been soaked in disinfectant solution. Allow the counter top to dry by evaporation. Wipe the outside surfaces of all supplies with wipes that have been soaked in disinfectant solution before placing the supplies into a class II biosafety cabinet. Wipe the surface of the Miltenyi rack and magnet with wipes that have been soaked in disinfectant solution before placing the Miltenyi supplies into a class II biosafety cabinet.

Harvest the cells as described above. Reconstitute the cells using SFD-BLSC-MACS buffer. Count the cells and divide the cells into 2 x 10⁶ cells per ml aliquots in 15 ml centrifuge tubes. Using sterile technique, add 1 pg per ml CD13 and 1 µg per ml CD90 for each 2 x 10⁶ cell aliquot. Vortex (Vortex Mixer^{™}) three times, using pulses of 1 second in length with a setting of 6. Incubate the cell aliquots for 60 minutes at ambient temperature.

Wash the cell carefully by adding 10-20x the volume of SFD-BLSC-MACS buffer (1st wash). Triturate the cell suspension 12-15 times. Centrifuge the tube(s) at 2000 x g for 5 minutes at ambient temperature. After centrifugation, discard the supernatant from the centrifuge tube by placing it in the bleach solution. Be sure to leave a small amount of the supernatant, about equal to the volume of the cellular pellet.

Resuspend the cellular pellet by raking the centrifuge tube across an 80-well microtube holder. Repeat this process 12-15 times. Wash the cells carefully by adding 10-20x the volume of SFD-BLSC-MACS buffer (2nd wash). Triturate the cell suspension 12-15 times. Centrifuge the tube(s) at 2000 x g for 5 minutes at ambient temperature. After centrifugation, discard the supernatant from the centrifuge tube by placing it in the bleach solution. Be sure to leave a small amount of the supernatant, about equal to the volume of the cellular pellet. Resuspend the cellular pellet by raking the centrifuge tube across an 80-well microtube holder. Repeat this process 12-15 times. Wash the cells carefully by adding 10-20x the volume of SFD-BLSC-MACS buffer (3rd wash). Triturate the cell suspension 12-15 times. Centrifuge the tube(s) at 2000 x g for 5 minutes at ambient temperature. After centrifugation, discard the supernatant from the centrifuge tube by placing it in the bleach solution. Be sure to leave approximately 200 µl of the supernatant. Resuspend the cellular pellet by raking the centrifuge tube across an 80-well microtube holder. Repeat this process 12-15 times.

Add 2 drops of the secondary antibody from Vector ABC kit (anti-mouse IgG-biotin, catalog #PK-4002, Vector Laboratories, Burlingame, CA). Vortex three times, using pulses of 1 second in length with a setting of 6. Incubate cell aliquots for 20 minutes at ambient temperature. Wash the cells carefully by adding 10-20x the volume of SFD-BLSC-MACS buffer (1st wash). Triturate the cell suspension 12-15 times. Centrifuge the tube(s) at 2000 x g for 5 minutes at ambient temperature. After centrifugation, discard the supernatant from the centrifuge tube by placing it in the bleach solution. Be sure to leave a small amount of the supernatant, about equal to the volume of the cellular pellet. Resuspend the cellular pellet by raking the centrifuge tube across an 80-well microtube holder. Repeat this process 12-15 times. Wash the cells carefully by adding 10-20x the volume of SFD-BLSC-MACS buffer (2nd wash). Triturate the cell suspension 12-15 times. Centrifuge the tube(s) at 2000 x g for 5 minutes at ambient temperature.

After centrifugation, discard the supernatant from the centrifuge tube by placing it in the bleach solution. Be sure to leave a small amount of the supernatant, about equal to the volume of the cellular pellet. Resuspend the cellular pellet by raking the centrifuge tube across an 80-well microtube holder. Repeat this process 12-15 times. Wash the cells carefully by adding 10-20x the volume of SFD-BLSC-MACS buffer (3rd wash). Triturate the cell suspension 12-15 times. Centrifuge the tube(s) at 2000 x g for 5 minutes at ambient temperature. After centrifugation, discard the supernatant from the centrifuge tube by placing it in the bleach solution. Be sure to leave approximately 200 µl of the supernatant of SFD-BLSC-MACS buffer per 10⁷ total cells. For fewer cells use same volume of buffer. Resuspend the cellular pellet by raking the centrifuge tube across an 80-well microtube holder. Repeat this process 12-15 times. Add 20 µl of MACS Anti-Biotin Microbeads (Catalog #130-090-485, Miltenyi Biotec Inc., Auburn, CA) per 10⁷ total cells. For fewer cells, use same volume. Vortex three times, using pulses of 1 second in length with a setting of 6. Incubate for 15 minutes at 6° to 12° C. Repeat the wash steps above and then resuspend the cell pellet in 500 µl of SFD-BLSC-MACS buffer per 10⁸ total cells. For fewer cells use same volume.

Choose a Miltenyi separation column (catalog #130-042-201, Miltenyi Biotec Inc.) for up to 2 x 10⁸ total cells. Place the column within the magnetic field. Prepare the column by washing it with 500 µl SFD-BLSC-MACS buffer. Collect the pass through volume in a tube and dispose of it by placing it in the bleach solution. Apply the cell suspension containing as many as to 10⁸ cells per 500 µl SFD-BLSC-MACS buffer onto the column (maximum column volume is 1000 µl. Allow the negative cells to pass through. Collect the pass through volume in a tube and keep it as the negative fraction. Rinse three times with 500 µl SFD-BLSC-MACS buffer. Add the rinse volumes to the negative fraction. Remove the column from the magnetic field.

Place the column on a suitable collection tube. Pipet I ml SFD-BLSC-MACS buffer onto the column. Remove the positive fraction by firmly pushing the SFD-BLSC-MACS buffer through the column using the plunger supplied with the column. Collect this volume in a tube as the positive fraction. Repeat this last process by adding 1 ml SFD-BLSC-MACS buffer to the column and pushing it through with the plunger. Centrifuge the cells and count the positive and negative fractions separately. The positive column fraction contains the germ layer lineage stem cells. The negative column fraction contains the pluripotent epiblastic-like stem cells and the totipotent blastomeric-like stem cells. Centrifuge the tubes containing the negative column fractions at 2000 x g for 5 minutes at ambient temperature. After centrifugation, discard the supernatant from the centrifuge tube by placing it in the bleach solution. Be sure to leave a small amount of the supernatant, about equal to the volume of the cellular pellet.

Resuspend the cellular pellet by raking the centrifuge tube across an 80-well microtube holder. Repeat this process 12-15 times. Reconstitute the cells in proliferation medium. Count, plate, and cultivate the cells.

Removal of pluripotent epiblastic-like stem cells: Put on gloves. Soak wipes with disinfectant solution. Wipe gloved hands with wipes that have been soaked in disinfectant solution. Wipe all the inside surfaces of a class II biosafety cabinet with wipes that have been soaked in disinfectant solution. Allow the surfaces to dry by evaporation. Wipe the outside counter top with wipes that have been soaked in disinfectant solution. Allow the counter top to dry by evaporation. Wipe the outside surfaces of all supplies with wipes that have been soaked in disinfectant solution before placing the supplies into a class II biosafety cabinet. Wipe the surface of the Miltenyi rack and magnet with wipes that have been soaked in disinfectant solution before placing the Miltenyi supplies into a class II biosafety cabinet.

Harvest the cells as described above. Reconstitute the cells using SFD-BLSC-MACS buffer buffer. Count the cells and divide the cells into 2 x10⁶ cells per ml aliquots in 15 ml centrifuge tubes. Using sterile technique, add 1 µg per ml CD10, 50 µg per ml SSEA-1, 50 µg per ml SSEA-3, and 50 µg per ml SSEA-4 for each 2 x 10⁶ cell aliquot. Vortex three times, using pulses of 1 second in length with a setting of 6. Incubate the cell aliquots for 60 minutes at ambient temperature.

Wash the cell carefully by adding 10-20x the volume of SFD-BLSC-MACS buffer (1st wash). Triturate the cell suspension 12-15 times. Centrifuge the tube(s) at 2000 x g for 5 minutes at ambient temperature. After centrifugation, discard the supernatant from the centrifuge tube by placing it in the bleach solution. Be sure to leave a small amount of the supernatant, about equal to the volume of the cellular pellet.

Resuspend the cellular pellet by raking the centrifuge tube across an 80-well microtube holder. Repeat this process 12-15 times. Wash the cells carefully by adding 10-20x the volume of SFD-BLSC-MACS buffer (2nd wash). Triturate the cell suspension 12-15 times. Centrifuge the tube(s) at 2000 x g for 5 minutes at ambient temperature. After centrifugation, discard the supernatant from the centrifuge tube by placing it in the bleach solution. Be sure to leave a small amount of the supernatant, about equal to the volume of the cellular pellet. Resuspend the cellular pellet by raking the centrifuge tube across an 80-well microtube holder. Repeat this process 12-15 times. Wash the cells carefully by adding 10-20x the volume of SFD-BLSC-MACS buffer (3rd wash). Triturate the cell suspension 12-15 times. Centrifuge the tube(s) at 2000 x g for 5 minutes at ambient temperature. After centrifugation, discard the supernatant from the centrifuge tube by placing it in the bleach solution. Be sure to leave approximately 200 µl of the supernatant. Resuspend the cellular pellet by raking the centrifuge tube across an 80-well microtube holder. Repeat this process 12-15 times.

Add 2 drops of the secondary antibody from Vector ABC kit (anti-mouse IgG-biotin, catalog #PK-4002, Vector Laboratories, Burlingame, CA). Vortex three times, using pulses of 1 second in length with a setting of 6. Incubate cell aliquots for 20 minutes at ambient temperature. Wash the cells carefully by adding 10-20x the volume of SFD-BLSC-MACS buffer (1st wash). Triturate the cell suspension 12-15 times. Centrifuge the tube(s) at 2000 x g for 5 minutes at ambient temperature. After centrifugation, discard the supernatant from the centrifuge tube by placing it in the bleach solution. Be sure to leave a small amount of the supernatant, about equal to the volume of the cellular pellet. Resuspend the cellular pellet by raking the centrifuge tube across an 80-well microtube holder. Repeat this process 12-15 times. Wash the cells carefully by adding 10-20x the volume of SFD-BLSC-MACS buffer (2nd wash). Triturate the cell suspension 12-15 times. Centrifuge the tube(s) at 2000 x g for 5 minutes at ambient temperature.

After centrifugation, discard the supernatant from the centrifuge tube by placing it in the bleach solution. Be sure to leave a small amount of the supernatant, about equal to the volume of the cellular pellet. Resuspend the cellular pellet by raking the centrifuge tube across an 80-well microtube holder. Repeat this process 12-15 times. Wash the cells carefully by adding 10-20x the volume of SFD-BLSC-MACS buffer (3rd wash). Triturate the cell suspension 12-15 times. Centrifuge the tube(s) at 2000 x g for 5 minutes at ambient temperature. After centrifugation, discard the supernatant from the centrifuge tube by placing it in the bleach solution. Be sure to leave approximately 200 µl of the supernatant of SFD-BLSC-MACS buffer per 10⁷ total cells. For fewer cells use same volume of buffer. Resuspend the cellular pellet by raking the centrifuge tube across an 80-well microtube holder. Repeat this process 12-15 times. Add 20 µl of MACS Anti-Biotin Microbeads (Catalog #130-090-485, Miltenyi Biotec Inc.) per 10⁷ total cells. For fewer cells, use same volume. Vortex three times, using pulses of 1 second in length with a setting of 6. Incubate for 15 minutes at 6° to 12° C. Repeat the wash steps above and then resuspend the cell pellet in 500 µl of SFD-BLSC-MACS buffer per 10⁸ total cells. For fewer cells use same volume.

Choose a Miltenyi separation column (catalog #130-042-201, Miltenyi Biotec Inc.) for up to 2 x 10⁸ total cells. Place the column within the magnetic field. Prepare the column by washing it with 500 µl SFD-BLSC-MACS buffer. Collect the pass through volume in a tube and dispose of it by placing it in the bleach solution. Apply the cell suspension containing as many as to 10⁸ cells per 500 µl SFD-BLSC-MACS buffer onto the column (maximum column volume is 1000 µl). Allow the negative cells to pass through. Collect the pass through volume in a tube and keep it as the negative fraction. Rinse three times with 500 µl SFD-BLSC-MACS buffer. Add the rinse volumes to the negative fraction. Remove the column from the magnetic field.

Place the column on a suitable collection tube. Pipet 1 ml SFD-BLSC-MACS buffer onto the column. Remove the positive fraction by firmly pushing the SFD-BLSC-MACS buffer through the column using the plunger supplied with the column. Collect this volume in a tube as the positive fraction. Repeat this last process by adding 1 ml SFD-BLSC-MACS buffer to the column and pushing it through with the plunger. Centrifuge the cells and count the positive and negative fractions separately. The positive column fraction contains the pluripotent epiblast-like stem cells. The negative column fraction contains the totipotent blastomere-like stem cells.

Centrifuge the negative column fraction tube(s) at 2000 x g for 5 minutes at ambient temperature. After centrifugation, discard the supernatant from the centrifuge tube by placing it in the bleach solution. Be sure to leave a small amount of the supernatant, about equal to the volume of the cellular pellet. Resuspend the cellular pellet by raking the centrifuge tube across an 80-well microtube holder. Repeat this step 12-15 times. Reconstitute the cells in SFD-BLSC Propagation medium. Count, plate, and cultivate the cells in suspension cultures. In numerous separate cultivations, so obtained BLSCs could be cultivated over more than 100, more typically over more than 200, and most typically over more than 300 doublings without loss of normal karyotype while preserving totipotent character. Remarkably, in additional experiments, the BLSC were continuously cultivated without spontaneous differentiation in defined serum-free medium and in the absence of differentiation inhibitors.

Repetitive single cell clonogenic analysis: Previous cloning studies (Young et al., 1993, 1998b, 2001 a, 2004b) revealed that repetitive single cell clonogenic analysis could be achieved if individual cells were grown in medium preconditioned by highly proliferating cells of the same parental line. Table 1 below depicts exemplary surface markers of the cells as separated:

**Table 1**

| -/- | BOUND | ELUANT |
|---|---|---|
| Size (plated) | Small | Very Small |
| Marker: | | |
| CEA-CAM-1 | - | + |
| SSEA-1 | + | - |
| SSEA-3 | + | - |
| SSEA-4 | + | - |
| CD66e | - | + |
| CD10 | + | - |
| Ectodermal | + | + |
| Mesodermal | + | + |
| Endodermal | + | + |
| Gametes | - | + |
| Identification | ELSC | BLSC |

Further analysis of molecules of interest found (in addition to telomerase) on BLSCs were CD66e, and CEA-CAM-1, and in several cases Oct-3/4, Nanong, Nanos, BMI-1, IDE1, IDE3, ABCG2, CXCR-4, and BCL-2, while SSEA-1, SSEA-3, SSEA-4, CD1 a, CD2, CD3, CD4, CD5, CD7, CDB, CD9, CD10, CD11 b, CD11 c, CD13, CD14, CD15, CD16, CD18, CD19, CD20, CD22, CD23, CD24, CD25, CD31, CD33, CD34, CD36, CD38, CD41, CD42b, CD45, CD49d, CD55, CD56, CD57, CD59, CD61, CD62E, CD65, CD68, CD69, CD71, CD79, CD83, CD90, CD95, CD105, CD106, CD117, CD123, CD135, CD166, Glycophorin-A, MHC-I, HLA-DRII, FMC-7, Annexin-V, and LIN cell surface markers were notably absent. Thus, isolated post-natal totipotent stem cells having surface markers CEA-CAM-1⁺, CD66e⁺, CD10⁻, SSEA-1⁻, SSEA-3⁻, and SSEA-4' were obtained in a relatively simple manner. Of course, it should be recognized that the so obtained cells may also be isolated from numerous other mammals, including rat, mouse, livestock, and human.

### Cloning

Put on gloves. Soak wipes with disinfectant solution. Wipe gloved hands with wipes that have been soaked in disinfectant solution. Wipe all the inside surfaces of a class II biosafety cabinet with wipes that have been soaked in disinfectant solution. Allow the surfaces to dry by evaporation. Wipe the outside counter top with wipes that have been soaked in disinfectant solution. Allow the counter top to dry by evaporation. Wipe the outside surfaces of all supplies with wipes that have been soaked in disinfectant solution before placing the supplies into a class II biosafety cabinet.

Remove cells from suspension cultures and process for cell counting, as described above. Dilute cells to clonal density: 1 cell per 5 µl of cloning medium. Using a 10 µl pipettor and 10 µl pipette tip, place the cell with 5 µl of medium in center of each well of Adult Stem Cell Coated 96-well plate (catalog # MBC-ASB-MSD-900-A011, Moraga Biotechnology Corp.). Add 50 µl of cloning medium (catalog # MBC-ASB-MED-100-A008, Moraga Biotechnology Corp.) to each well. Wait six hours, and then count the number of cells in each well. For wells having no cells or two or more cells, remove the medium. Incubate wells having no cells or two or more cells with 100 µl of 70% ethanol for 10 minutes. Replace the alcohol with 200 µl of 5% (v/v) sodium azide solution in sterile rinse buffer. Check the wells every three days for cell growth. When the proliferating cells reach approximately 50% of cells suspended in medium, add 50 µl of cloning medium. When the proliferating cells reach approximately 70% of cells suspended in medium, add 100 µl of cloning medium. When the proliferating cells reach 90% of confluence, remove the cells from the wells by pipetting cell suspension. Add cell suspension *in toto* into adult stem cell-coated 24 well plates and feed them with 0.5 ml of cloning medium. When the proliferating cells reach approximately 50% of cells suspended in medium, add 0.5 ml of cloning medium. When the cells reach approximately 70% of cells suspended in medium, add 1.0 ml of cloning medium. When the cells reach 90% of confluence, remove the cells from the wells by pipetting cell suspension. Add cell suspension *in toto* into adult stem cell-coated 6 well plates and feed them with 1.0 ml of cloning medium. When the proliferating cells reach approximately 50% of cells suspended in medium, add 1.0 ml of cloning medium. When the approximately 70% of cells suspended in medium, add 2.0 ml of cloning medium. When the cells reach 90% of confluence, remove the cells from the wells by pipetting cell suspension. Add cell suspension *in toto* into adult stem cell-coated 25 cm² flask. Add SFD-BLSC propagation medium to the flasks every 24-48 hours, depending on the percentage of cells within the flask(s). [For example, when the approximate percentage of the cells in the flask is less than 50%, feed the culture(s) with 5 ml per 25 cm² flask of SFD-BLSC propagation medium. When the approximate percentage of the cells in the flask is 60-70%, feed the culture(s) with 10-15 ml per 25 cm² flask of SFD-BLSC propagation medium. Once the approximate percentage of the cells in the flask is greater than 75%, harvest the cells and divide into new flasks with a starting inoculation density of 0.5 x 10⁶ cells per 5 ml of SFD-BLSC Propagation medium for 25 cm² gelatin-coated flasks or 1.0 x 10⁶ cells per 10 ml of SFD-BLSC Propagation medium for 75 cm² gelatin-coated flasks.

### Lineage Induction of Blastomere-Like Stem Cells

Induced phenotypes: Adult blastomere-like stem cells can be induced into downstream lineages, e.g., germ cells and placental tissues, epiblast-like stem cells, ectodermal germ layer lineage stem cells, mesodermal germ layer lineage stem cells, and endodermal germ layer lineage stem cells utilizing general and/or specific induction media. The specific description given below is representative of exemplary induction strategies.

Put on gloves. Soak wipes with disinfectant solution. Wipe gloved hands with wipes that have been soaked in disinfectant solution. Wipe all the inside surfaces of a class II biosafety cabinet with wipes that have been soaked in disinfectant solution. Allow the surfaces to dry by evaporation. Wipe the outside counter top with wipes that have been soaked in disinfectant solution. Allow the counter top to dry by evaporation. Wipe the outside surfaces of all supplies with wipes that have been soaked in disinfectant solution before placing the supplies into a class II biosafety cabinet.

Remove cells from suspension cultures. Prepare sterile 15-ml polypropylene centrifuge tubes by wiping outside with disinfectant agent and placing in sterile Class II Biosafety cabinet. Under sterile conditions remove 14 ml cell suspension from tissue culture flask and place into each tube. This can be accomplished by either pouring or pipetting the cell suspension from the flask into the tube(s). Centrifuge the 15-ml polypropylene tubes at 2000 x g for 5 minutes at ambient temperature. After centrifugation, discard the supernatant from the centrifuge tube by placing it in the bleach solution. Be sure to leave a small amount of the supernatant, about equal the volume of the cell pellet. Resuspend the cell pellet by raking the centrifuge tubes across an 80-well microtube holder. Repeat this process 12-15 times.

Use a 5 ml pipette to wash and triturate each 15 ml centrifuge tube in sequence. Use 1-5 ml of general induction medium, pH 7.4 (catalog # MBC-ASB-IMDG-100-A001, Moraga Biotechnology Corp.) in this process. The volume to be used will depend upon the volume of the cell suspension to be resuspended. Place the combined cell suspension in a 15 ml tube. Count the cells as outlined above.

Reconstitute cells at 5 x 10³ cells per ml in general induction medium and aliquot 200 µl of cell suspension into each well of a 96-well adult stem ell coated culture plate (catalog # MBC-ASB-MSD-900-A011, Moraga Biotechnology Corp.). Final cell concentration will be 10³ cells per well. Place the plate(s) into a humidified incubator that uses an environment of 95% air/5% carbon dioxide and is set at 37° C. After 48 hr incubation the medium is removed, the cells rinsed twice with 150 µl of sterile serum-free defined-BLSC rinse buffer, Ca⁺²/Mg⁺², pH 7.4 (catalog # MBC-ASB-REC-100-A001, Moraga Biotechnology Corp.), the rinse solution removed and replaced with the appropriate induction media (see below) dependent on cell(s) of interest. The cultures are fed every other day with an exchange of culture medium, i.e., 150 µl of spent medium is aspirated from each well and 150 µl of the appropriate fresh induction medium is added to each well. The cultures are returned to the 95% air/5% CO₂ humidified 37°C environment after medium exchange for further culturing.

Induction media: The following general and specific induction media are utilized to engender germ cells and placental tissues, epiblast-like stem cells, ectodermal germ layer lineage stem cells, mesodermal germ layer lineage stem cells, and endodermal germ layer lineage stem cells. General induction medium, pH 7.4 (catalog # MBC-ASB-IMDG-100-A001, Moraga Biotechnology Corp.) will non-specifically induce all aforementioned cell types, e.g., germ cells and placental tissues, epiblast-like stem cells, ectodermal germ layer lineage stem cells, mesodermal germ layer lineage stem cells, and endodermal germ layer lineage stem cells, with respective phenotypic expression markers indicative of specific cell types appearing within the cells from 7-70 days after plating. Ectodermal induction medium, pH 7.4 (catalog # MBC-ASB-IMIDE-100-A002, Moraga Biotechnology Corp.) will engender cells of the ectodermal lineage, e.g., neuronal-associated cells and epidermal-associated cells, with respective phenotypic expression markers indicative of specific cell types appearing within the cells from 14-56 days after plating. Mesodermal induction medium, pH 7.4 (catalog # MBC-ASB-IMDM-100-A003, Moraga Biotechnology Corp.) will engender cells of the mesodermal lineage, e.g., muscle, fat, cartilage, bone, connective tissue, dermis, blood cells, endothelial cells, etc., with respective phenotypic expression markers indicative of specific cell types appearing within the cells from 7-70 days after plating. Endodermal induction medium, pH 7.2 (catalog # MBC-ASB-IMDN-100-A004, Moraga Biotechnology Corp.) will engender cells of the endodermal lineage, e.g., gastrointestinal epithelial cells, liver cells, pancreas cells, etc., with respective phenotypic expression markers indicative of specific cell types appearing within the cells from 7-70 days after plating.

Thus, specific embodiments and applications of non-embryonic totipotent blastomere-like stem cells have been disclosed. It should be apparent, however, to those skilled in the art that many more modifications besides those already described are possible. Moreover, in interpreting both the specification and the claims, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced. Furthermore, where a definition or use of a term in a reference is inconsistent or contrary to the definition of that term provided herein, the definition of that term provided herein applies and the definition of that term in the reference does not apply.

## Claims

1. An isolated rat post-natal blastomere-like stem cell (BLSC) having a size of less than 1 µm, expressing surface marker CEA-CAM-1, and not expressing surface markers SSEA-1, SSEA-3, and SSEA-4 and being trypan-positive.

2. The stem cell of claim 1 wherein the stem cell is a mammalian cell having surface marker CD66e and not CD10.

3. The stem cell of claim 1 wherein the cell has potency to differentiate into a placental cell or a germ cell upon stimulation with an induction medium.

4. The stem cell of claim 1 wherein the cell has potency to differentiate into an epiblast-like stem cell upon stimulation with an induction medium.

5. The stem cell of claim 1 wherein the cell has potency to differentiate into an ectodermal germ layer lineage stem cell upon stimulation with an ectodermal-specific induction medium.

6. The stem cell of claim 1 wherein the cell has potency to differentiate into a mesodermal germ layer lineage stem cell upon stimulation with a mesodermal-specific induction medium.

7. The stem cell of claim 1 wherein the cell has potency to differentiate into an endodermal germ layer lineage stem cell upon stimulation with an endodermal-specific induction medium.

8. The stem cell of claim 1 wherein the cell undergoes at least 100 doublings while maintaining totipotent character in a serum-free defined propagation medium in the absence of differentiation inhibitors.

9. The stem cell of claim 1 wherein the cell undergoes at least 300 doublings while maintaining totipotent character in a serum-free defined propagation medium in the absence of differentiation inhibitors.

10. The stem cell of claim 1 wherein the cell does not spontaneously differentiate in serum-free defined propagation medium in the absence of differentiation inhibitors.

11. The stem cell of claim 1 wherein the cell remains quiescent when implanted into an animal and does not form a cancerous tissue.

12. The stem cell of claim 1 wherein the cell differentiates in an animal having tissue damage and does not form a cancerous tissue.

13. The stem cell of claim 1 wherein the cell expresses at least one of telomerase, Oct-3/4, Nanog, Nanos, BMI-1, IDE1, IDE3, ABCG2, CXCR-4, and BCL-2, and wherein the cell does not express at least one of CD1 a, CD2, CD3, CD4, CD5, CD7, CDB, CD9, CD11 b, CD11 c, CD13, CD14, CD15, CD16, CD18, CD19, CD20, CD22, CD23, CD24, CD25, CD31, CD33, CD34, CD36, CD38, CD41, CD42b, CD45, CD49d, CD55, CD56, CD57, CD59, CD61, CD62E, CD65, CD68, CD69, CD71, CD79, CD83, CD90, CD95, CD105, CD106, CD117, CD123, CD135, CD166, Glycophorin-A, MHC-I, HLA-DRII, FMC-7, Annexin-V, and LIN.

14. The stem cell of claim 1 wherein the cell expresses CEA-CAM-1, and telomerase, and wherein the cell does not express MHC-I.

15. A method of isolating a stem cell according to claim 1 comprising:
(i) receiving a plurality of cells from a rat tissue;
(ii) cultivating the cells past confluence to obtain multiple confluent layers, and collecting the cultivated cells;
(iii) slow-freezing the plurality of cells to a temperature of higher than -100°C for at least 12 hours and thawing the cells thereafter;
(iv) removing germ line layer stem cells and epiblast-like stem cells from the thawed cells using cell surface markers to form a cell suspension such that the suspension is enriched in stem cells having surface markers CEA-CAM-1⁺, SSEA-1⁻, SSEA-3⁻, and SSEA-4⁻.

16. The method of claim 15 wherein the rat tissue is connective tissue, and wherein the stem cells have the surface markers CD66e⁺ and CD10⁻.

17. The method of claim 15 wherein the germ layer lineage stem cells are removed using antibodies specific to at least one of CD13 and CD90.

18. The method of claim 15 wherein the epiblast-like stem cells are removed using antibodies specific to at least one of CD10, SSEA-1, SSEA-3, and SSEA-4.

19. The method of claim 15 wherein the thawed cells are cultivated to increase the number of cells before the step of removing the germ layer lineage stem cells and the epiblast-like stem cells.

20. The method of claim 15 further comprising a step of cloning the stem cells having surface markers CEA-CAM-1⁺, CD66e⁺, CD10⁻, SSEA-1⁻, SSEA-3⁻, and SSEA-4⁻ to thereby obtain monoclonal populations of the stem cells according to claim 1.

## Patentansprüche

1. Isolierte postnatale Blastomer-ähnliche Säuger-/Human- Stammzelle (BLSC) mit einer Größe von weniger als 1µm, die den Oberflächenmarker CEA-CAM-1 exprimiert und die Oberflächenmarker SSEA-1, SSEA-3 und SSEA-4 nicht exprimiert und Trypan-positiv ist.

2. Stammzelle nach Anspruch 1, wobei die Stammzelle eine Säugerstammzelle ist, die den Oberflächenmarker CD66e und nicht CD10 aufweist.

3. Stammzelle nach Anspruch 1, wobei die Zelle die Fähigkeit hat, nach Stimulation mit einem Induktionsmedium in eine Plazentazelle oder eine Keimzelle zu differenzieren.

4. Stammzelle nach Anspruch 1, wobei die Zelle die Fähigkeit hat, nach Stimulation mit einem Induktionsmedium in eine Epiblasten-ähnliche Stammzelle zu differenzieren.

5. Stammzelle nach Anspruch 1, wobei die Zelle die Fähigkeit hat, nach Stimulation mit einem Ektoderm-spezifischen Induktionsmedium in eine vom ektodermalen Keimblatt abstammende Stammzelle zu differenzieren.

6. Stammzelle nach Anspruch 1, wobei die Zelle die Fähigkeit hat, nach Stimulation mit einem Mesoderm-spezifischen Induktionsmedium in eine vom mesodermalen Keimblatt abstammende Stammzelle zu differenzieren.

7. Stammzelle nach Anspruch 1, wobei die Zelle die Fähigkeit hat, nach Stimulation mit einem Endoderm-spezifischen Induktionsmedium in eine vom endodermalen Keimblatt abstammende Stammzelle zu differenzieren.

8. Stammzelle nach Anspruch 1, wobei sich die Zelle in einem serumfreien, definierten Verbreiterungsmedium in Abwesenheit von Differenzierungsinhibitoren mindestens 100 mal unter Beibehaltung des totipotenten Charakters verdoppelt.

9. Stammzelle nach Anspruch 1, wobei sich die Zelle in einem serumfreien, definierten Verbreiterungsmedium in Abwesenheit von Differenzierungsinhibitoren mindestens 300 mal unter Beibehaltung des totipotenten Charakters verdoppelt.

10. Stammzelle nach Anspruch 1, wobei die Zelle in serumfreiem, definiertem Verbreiterungsmedium in Abwesenheit von Differenzierungsinhibitoren nicht spontan differenziert.

11. Stammzelle nach Anspruch 1, wobei die Zelle, wenn sie in ein Tier implantiert wird, ruhend bleibt und kein kanzeröses Gewebe bildet.

12. Stammzelle nach Anspruch 1, wobei die Zelle in einem Tier, das Gewebeschäden hat, differenziert und kein kanzeröses Gewebe bildet.

13. Stammzelle nach Anspruch 1, wobei die Zelle mindestens eines von Telomerase, Oct-3/4, Nanog, Nanos, BMI-1, IDE1, IDE3, ABCG2, CXCR-4 und BCL-2 exprimiert und wobei die Zelle nicht mindestens eines von CD1 a, CD2, CD3, CD4, CD5, CD7, CDB, CD9, CD11 b, CD11 c, CD13, CD14, CD15, CD16, CD18, CD 19, CD20, CD22, CD23, CD24, CD25, CD31, CD33, CD34, CD36, CD38, CD41, CD42b, CD45, CD49d, CD55, CD56, CD57, CD59, CD61, CD62E, CD65, CD68, CD69, CD71, CD79, CD83, CD90, CD95, CD105, CD106, CD 117, CD123, CD135, CD166, Glycophorin-A, MHC-I, HLA-DRII, FMC-7, Annexin-V und LIN exprimiert.

14. Stammzelle nach Anspruch 1, wobei die Zelle CEA-CAM-1 und Telomerase exprimiert, und wobei die Zelle nicht MHC-I exprimiert.

15. Verfahren zur Isolierung von Stammzellen nach Anspruch 1, bei dem:
(i) eine Vielzahl von Zellen aus einem Säuger-/Humangewebe erhalten wird;
(ii) die Zellen über Konfluenz hinaus kultiviert werden, um mehrfachkonfluente Schichten zu erhalten, und die kultivierten Zellen gesammelt werden;
(iii) die Vielzahl von Zellen langsam auf eine Temperatur von höher als - 100°C für mindestens 12 Stunden eingefroren werden und die Zellen danach aufgetaut werden;
(iv) die Keimblattlinienstammzellen und Epiblasten-ähnlichen Stammzellen unter Verwendung von Zelloberflächenmarkern von den aufgetauten Zellen derart unter Bildung einer Zellsuspension abgetrennt werden, dass die Suspension mit Stammzellen angereichert ist, welche die Oberflächenmarker CEA-CAM-1⁺, SSEA-1⁻, SSEA-3⁻ und SSEA-4⁻ aufweisen.

16. Verfahren nach Anspruch 15, wobei das Säugergewebe Bindegewebe ist und wobei die Stammzellen die Oberflächenmarker CD66e⁺ und CD10⁻ aufweisen.

17. Verfahren nach Anspruch 15, wobei die vom Keimblatt abstammenden Stammzellen unter Verwendung von Antikörpern abgetrennt werden, die für mindestens eines von CD 13 und CD90 spezifisch sind.

18. Verfahren nach Anspruch 15, wobei die Epiblasten-ähnlichen Stammzellen unter Verwendung von Antikörpern abgetrennt werden, die für mindestens eines von CD10, SSEA-1, SSEA-3 und SSEA-4 spezifisch sind.

19. Verfahren nach Anspruch 15, wobei die aufgetauten Zellen kultiviert werden, um vor dem Schritt, in dem die vom Keimblatt abstammenden Stammzellen und die Epiblasten-ähnlichen Stammzellen abgetrennt werden, die Anzahl der Zellen zu erhöhen.

20. Verfahren nach Anspruch 15, das weiterhin einen Schritt umfasst, in dem Stammzellen mit den Oberflächenmarkern CEA-CAM-1⁺, CD66e⁺, CD10⁻, SSEA-1⁻ SSEA-3⁻ und SEA-4⁻ geklont werden, um **dadurch** monoklonale Populationen der Stammzellen nach Anspruch 1 zu erhalten.

## Revendications

1. Une cellule souche isolée postnatale mammifère/humaine de type blastomère (BLSC) ayant une taille inférieure à 1 µm, exprimant le marqueur de surface CEA-CAM-1, et n'exprimant pas les marqueurs de surface SSEA-1, SSEA-3, et SSEA-4 et étant positive au trypan.

2. La cellule souche selon la revendication 1, la cellule souche étant une cellule de mammifère ayant le marqueur de surface CD66e et non CD 10.

3. La cellule souche selon la revendication 1, la cellule souche ayant la capacité de se différencier en une cellule placentaire ou une cellule germinale via stimulation avec un milieu d'induction.

4. La cellule souche selon la revendication 1, la cellule souche ayant la capacité de se différencier en une cellule souche de type épiblaste via stimulation avec un milieu d'induction.

5. La cellule souche selon la revendication 1, la cellule souche ayant la capacité de se différencier en une cellule souche de lignage de feuillet embryonnaire ectodermique via stimulation avec un milieu d'induction spécifique ectodermique.

6. La cellule souche selon la revendication 1, la cellule souche ayant la capacité de se différencier en une cellule souche de lignage de feuillet embryonnaire mésodermique via stimulation avec un milieu d'induction spécifique mésodermique.

7. La cellule souche selon la revendication 1, la cellule souche ayant la capacité de se différencier en une cellule souche de lignage de feuillet embryonnaire endodermique via stimulation avec un milieu d'induction spécifique endodermique.

8. La cellule souche selon la revendication 1, la cellule subissant au moins 100 duplications tout en maintenant le caractère totipotent dans un milieu de propagation défini sans sérum en l'absence d'inhibiteurs de différenciation.

9. La cellule souche selon la revendication 1, la cellule subissant au moins 300 duplications tout en maintenant le caractère totipotent dans un milieu de propagation défini sans sérum en l'absence d'inhibiteurs de différenciation.

10. La cellule souche selon la revendication 1, la cellule ne se différenciant pas spontanément dans un milieu de propagation défini sans sérum en l'absence d'inhibiteurs de différenciation.

11. La cellule souche selon la revendication 1, la cellule restant quiescente lorsqu'elle est implantée dans un animal et ne formant pas un tissu cancéreux.

12. La cellule souche selon la revendication 1, la cellule souche se différenciant dans un animal ayant un dommage du tissu et ne formant pas un tissu cancéreux.

13. La cellule souche selon la revendication 1, la cellule exprimant au moins un élément parmi télomérase, Oct-3/4, Nanog, Nanos, BMI-1, IDE1, IDE3, ABCG2, CXCR-4, et BCL-2, et la cellule n'exprimant pas au moins un élément parmi CD1 a, CD2, CD3, CD4, CD5, CD7, CDB, CD9, CD11 b, CD11 c, CD 13, CD 14, CD 15, CD16, CD18, CD19, CD20, CD22, CD23, CD24, CD25, CD31, CD33, CD34, CD36, CD38, CD41, CD42b, CD45, CD49d, CD55, CD56, CD57, CD59, CD61, CD62E, CD65, CD68, CD69, CD71, CD79, CD83, CD90, CD95, CD105, CD106, CD117, CD123, CD 13 5, CD166, glycophorine-A, MHC-I, HLA-DRII, FMC-7, annexine V, et LIN.

14. La cellule souche selon la revendication 1, la cellule exprimant CEA-CAM-1, et télomérase, et la cellule n'exprimant pas MHC-I.

15. Un procédé d'isolation d'une cellule souche selon la revendication 1 comprenant les étapes suivantes :
(i) réceptionner une pluralité de cellules à partir d'un tissu mammifère/humain ;
(ii) cultiver la confluence passée des cellules pour obtenir des couches confluentes multiples, et collecter les cellules cultivées ;
(iii) congeler lentement la pluralité de cellules à une température de plus de -100°C pour au moins 12 heures et décongeler les cellules par la suite ;
(iv) enlever des cellules souches de feuillet de lignée embryonnaire et des cellules souches de type épiblaste des cellules décongelées en utilisant des marqueurs de surface cellulaire ou marqueurs antigéniques pour former une suspension cellulaire de sorte que la suspension est enrichie en cellules souches ayant des marqueurs de surface CEA-CAM-1⁺, SSEA-1⁻, SSEA-3⁻, et SSEA-4⁻.

16. Le procédé selon la revendication 15, dans lequel le tissu mammifère est du tissu conjonctif, et dans lequel les cellules souches ont les marqueurs de surface CD66e⁺ et CD10⁻.

17. Le procédé selon la revendication 15, dans lequel les cellules souches de lignage de feuillet embryonnaire sont enlevées en utilisant des anticorps spécifiques pour au moins un des éléments CD13 et CD90.

18. Le procédé selon la revendication 15, dans lequel les cellules souches de type épiblaste sont enlevées en utilisant des anticorps spécifiques pour au moins un des éléments CD10, SSEA-1, SSEA-3, et SSEA-4.

19. Le procédé selon la revendication 15, dans lequel les cellules décongelées sont cultivées pour augmenter le nombre de cellules avant l'étape consistant à enlever les cellules souches de lignage de feuillet embryonnaire et les cellules souches de type épiblaste.

20. Le procédé selon la revendication 15, comprenant en outre une étape consistant à cloner les cellules souches ayant les marqueurs de surface CEA-CAM-1⁺, CD66e⁺, CD10⁻, SSEA-1⁻, SSEA-3⁻, et SSEA-4⁻ pour obtenir ainsi des populations monoclonales des cellules souches selon la revendication 1.
